(19) Europäisches Patentamt

European Patent Office

Office européen des brevets

(11) **EP 3 787 700 B1**

(12) **EUROPEAN PATENT SPECIFICATION**

(45) Date of publication and mention
of the grant of the patent:
**28.02.2024   Bulletin 2024/09**

(21) Application number: **19720681.6**

(22) Date of filing: **03.05.2019**

(51) International Patent Classification (IPC):
**A61L 27/12** *(2006.01)*      **B29C 64/106** *(2017.01)*

(52) Cooperative Patent Classification (CPC):
**A61L 27/12; B33Y 10/00;** A61L 2400/06;
B33Y 80/00

(86) International application number:
**PCT/EP2019/061403**

(87) International publication number:
**WO 2019/211449 (07.11.2019 Gazette 2019/45)**

(54) **SYNTHETIC BONE GRAFT**

SYNTHETISCHES KNOCHENIMPLANTAT

GREFFE OSSEUSE SYNTHÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **03.05.2018   EP 18382309**

(43) Date of publication of application:
**10.03.2021   Bulletin 2021/10**

(73) Proprietor: **Mimetis Biomaterials
08025 Barcelona (ES)**

(72) Inventors:
• **MAAZOUZ, Yassine
08290 Cerdanyola del Vallès (ES)**
• **RAYMOND LLORENS, Santiago
08290 Cerdanyola del Vallès (ES)**
• **GINEBRA MOLINS, Maria-Pau
08290 Cerdanyola del Vallès (ES)**

(74) Representative: **Jensen, Olaf Sven
Nobel Biocare Services AG
Intellectual Property Department
Balz-Zimmermann-Strasse 7
8302 Kloten (CH)**

(56) References cited:
**US-A1- 2016 082 156**

• **YASSINE MAAZOUZ ET AL: "Self-hardening and
thermoresponsive alpha tricalcium
phosphate/pluronic pastes", ACTA
BIOMATERIALIA, vol. 49, 1 February 2017
(2017-02-01), pages 563-574, XP055519918,
AMSTERDAM, NL ISSN: 1742-7061, DOI:
10.1016/j.actbio.2016.11.043**
• **S. MICHNA; W. WU; J. A. LEWIS: "Concentrated
hydroxyapatite inks for direct-write assembly of
3-D periodic scaffolds", BIOMATERIALS, vol. 26,
no. 28, October 2005 (2005-10), pages 5632-5639,
XP027767516, ISSN: 0142-9612, DOI:
10.1016/j.biomaterials.2005.02.040 cited in the
application**

## Description

### Technical field

The present invention relates to the field of bone graft substitutes. In particular, the invention provides a method for producing a synthetic bone graft substitute by 3D printing, an ink used for this method as well as the synthetic bone grafts.

### Background art

The recent developments in three dimensional (3D) printing technologies have opened vast opportunities for the development of patient-specific bone graft substitutes, which is expected to have a particularly high impact on dental and maxillofacial surgery as well as in trauma and orthopaedic surgery. Whereas autologous or allogenic bone grafts are still the gold standard in the frequent bone grafting surgical procedures performed nowadays,[1,2] the possibility to fabricate customised scaffolds based on digitalised medical imaging techniques has provided a real boost to the applications of synthetic bone grafts. The novel fabrication approaches referred to as 3D printing techniques are based on computer-aided design (CAD) and computer-aided manufacturing (CAM) and result in a patient-specific bone grafting therapy. Such technologies allow the design of personalised synthetic bone grafts, matching the specificity of every defect in terms of both the metabolic and anatomic characteristics, opening new perspectives in this field.[3]

Previous works reported the use of such techniques employing polymers, ceramics, or even metal alloys.[4,5] In the case of bioceramics, the works revolve around either powder bed printing, which consists in dispensing a liquid binder or reactive fluid onto a powder bed in a layer by layer process,[6] or in filament-based direct ink writing (DIW), also referred to as robocasting, microextrusion or three-dimensional plotting [7] which consists in dispensing layer by layer an ink composed of ceramic particles homogenously dispersed in a binder following an *in silica* designed pattern. The main advantage of filament-based direct ink writing over powder bed printing is that there is no need to clean the printed object from remaining particles from the bed that can provoke adverse inflammatory responses of the host tissue. The design of the inks plays a central part in this technology. They must present a pseudoplastic behavior in order to withstand the weight of the superimposed layers and must be completely injectable, allowing a continuous printing.[8] Furthermore, by tailoring the ink composition it is possible to tune the physicochemical properties of the scaffolds in terms of chemistry, textural properties, and multiscale porosity.

The use of DIW with calcium phosphate (CaP)-based inks for the fabrication of bone implants and scaffolds for bone tissue engineering was proposed for the first time in 2005.[9] Several works have reported the fabrication and properties of robocasted scaffolds made of hydroxyapatite (HA),[9-11] beta-tricalcium phosphate (β-TCP) [12,13], biphasic combinations of β-TCP and HA [11,14-17] and bioactive glasses.[18-24]

Nonetheless, in all these pioneer works, after DIW the samples had to be consolidated by sintering at high temperature. The effects of sintering conditions on the chemical, structural and mechanical properties of those samples have been deeply studied.[11,16,25,26]

Recently, the possibility to fabricate low-temperature self-setting inks that do not require the sintering step has been proposed, using formulations based on beta-calcium silicate (β-CaSiO₃),[20,27] magnesium phosphates[28] or calcium phosphates.[29,30] Most calcium phosphate self-setting inks are based on alpha-tricalcium phosphate (α-TCP), which can be combined with different binders, like gelatine,[29] type I collagen[31] hydroxypropyl methylcellulose (HPMC),[32] a mixture of Polysorbate 80 (Tween 80), short-chain triglycerides (Miglyol 812) and phosphoric acid monohexadecyl ster,[30,34] polyvinyl alcohol[33] and alginic acid.[33] The hardening process of these inks is due to the hydrolysis of α-TCP to a calcium deficient hydroxyapatite (CDHA), resulting in nano/micro structured crystals that are much closer to the mineral phase of bone than sintered CaPs.

However, while the low-temperature chemical consolidation of these scaffolds makes it possible to obtain a biomimetic product, closer to bone mineral than the high-temperature sintered scaffolds, the binders used in the previously described self-setting inks are difficult to remove after hardening or are even intended to remain in the synthetic bone graft. Consequently, these scaffolds differ from the natural mineral phase of bone. As residual binder can potentially interfere with the remodeling process of the ceramic, its presence is undesired.

Maazouz et al. (acta biomaterialia, vol. 49, pages 563-574, doi : 10.1016/j.actbio.2016.11.043) discloses an injectable composition (cement) comprising alpha TCP and poloxamer as binder. after realization of the scaffold by extrusion, the TCP is hydrolysed to obtained Ca deficient Hap.

It is desirable to provide a scaffold that largely resembles the natural mineral phase of bone for bone formation (osteoconduction) upon implantation. The present invention thus addresses the problem of providing such a bone graft.

## Summary of the invention

The inventors of the present invention have surprisingly found that it is possible to produce a synthetic bone graft formed of a scaffold having a mineral phase closer to bone than prior art scaffolds which are consolidated by sintering at high temperatures. This may be achieved by low-temperature consolidation of self-setting inks comprising a binder. Additionally, the inventors found that under certain processing conditions it is possible to remove a large proportion of the binder from the scaffold during the hardening processes. The resulting scaffold has a mineral phase closer to bone and a minimal amount of binder, which makes the scaffold more biocompatible. The scaffold may be used as a synthetic bone graft.

The present invention thus relates to a 3D-printed synthetic bone graft as defined in claim 1.

The calcium-deficient hydroxyapatite is present in an amount of at least 60 wt.%, preferably at least 70 wt.%, preferably at least 80 wt.%, preferably at least 90 wt.% with respect to the total weight of the composition.

The present invention thus relates to a synthetic bone graft with a composition comprising calcium-deficient hydroxyapatite (CDHA) in an amount of at least 60 wt.% with respect to the total weight of the composition, optionally $\alpha$-TCP and/or $\beta$-TCP.

The composition of the synthetic bone graft further comprises a binder. The binder is present in 5 wt.% or less with respect to the total weight of the composition. The binder may be present in the range of 0.25 wt.% to 5 wt.% with respect to the total weight of the composition, preferably in the range of 0.25 wt.% to 1 wt.% with respect to the total weight of the composition. The binder may be a poly(oxypropylene)-poly(oxyethylene) copolymer, such as poloxamer, for example Poloxamer 407 or Poloxamer P88. The composition has a macro-porosity in the range of 10% to 60%, as determined by calculating the difference between the total porosity and the nanomacroporosity is defined as a per entrance size larger than 10 microns, the pore entrance size being determined by mercury intrusion porosimetry.

The calcium-deficient hydroxyapatite in the composition of the synthetic bone graft may be doped with one or more ions. The ions may be selected from anions such as carbonate, bicarbonate, silicate, and/or cations such as Ca, Mg, Sr, Ce, Al, Zn, Ag, Co, Cu and other transition metals.

The composition preferably shows a needle-like morphology as determined by Field Emission-Scanning Electron Microscopy.

The composition of the synthetic bone graft may have a compressive strength of 2 to 12 MPa as determined by monotonic compressive loading of cylinders of 6 mm of diameter and 12 mm of height (6x12 mm).

The composition of the synthetic bone graft may have a total porosity in the range of 20 % to 80%, as determined by mercury intrusion porosimetry.

The composition of the synthetic bone graft may have pores of less than 1 $\mu$m, as determined by mercury intrusion porosimetry.

The composition of the synthetic bone graft may have a skeletal density in the range of 2.65 to 3.14 g/cm$^3$, preferably in the range of 2.70 to 3.14 g/cm$^3$, as determined by helium pycnometry. Furthermore, the present invention provides a self-setting ink as defined in claim 9 useful for the manufacture of the synthetic bone graft of the invention. This self-setting ink comprises $\alpha$-TCP powder and a binder solution. The binder solution preferably comprises a setting medium (such as water) and a poly(oxypropylene)-poly(oxyethylene) copolymer (such as a poloxamer).

Preferably the self-setting ink (essentially) consists of $\alpha$-TCP powder and a binder solution comprising a poloxamer and water.

The binder solution in the self-setting ink contains 25g to 60g poloxamer per 100 ml solution. The binder solution in the self-setting ink may contain more than 30 to 60 g poloxamer per 100 ml solution, preferably 35 to 60g poloxamer per 100 ml solution. Preferably, the binder solution in the self-setting ink may contain 30 g poloxamer per 100 ml solution. The binder solution in the self-setting ink may contain from 25g to less than 30g poloxamer per 100 ml solution or more than 30 to 60 g poloxamer per 100 ml solution.

An amount of 0.3 to 0.7 g of binder solution may be used per g of $\alpha$-TCP powder.

The setting medium can be water or an aqueous solution containing inorganic ions.

The present invention also provides a method for producing a synthetic bone graft as defined in claim 12, comprising the
following steps:

> a. providing a self-setting ink comprising $\alpha$-TCP powder and a binder solution made of a poly(oxypropylene)-poly(oxyethylene) copolymer (such as a poloxamer) and water;
> b. 3D printing to provide a scaffold
> c. placing the scaffold in a humid atmosphere; and
> d. hardening the scaffold by immersion in water or an aqueous solution consisting of water and one or more ions selected from the group consisting of anions such as carbonate, bicarbonate, silicate, and/or cations such as Ca, Mg, Sr, Ce, Al, Zn, Ag, Co, Cu, and other transition metals.

Preferably the one or more ions are selected from the group consisting of carbonate, bicarbonate, silicate, calcium, strontium, zinc, cobalt and copper.

These ions can be incorporated in the CDHA during its precipitation while the process of hydrolysis of $\alpha$-TCP occurs. This way, the ion doping process is simultaneous to the CDHA formation, and it allows the shape and geometry of the printed scaffold to be maintained.

In step b) the 3D printing process is performed to provide a shape that has been pre-determined by a digitalised medical imaging technique and/or computer aided design.

In step c) (which is also referred to as the "pre-setting step") the relative humidity of the humid atmosphere may be in the range of 70%-100%, preferably in the range of 90%-100%. The scaffold may be heated at a temperature in the range of 30 to 100°C, preferably 50 to 100°C. The scaffold may be heated at a temperature in the range of 30-50°C, preferably 30-40 °C. The scaffold may be exposed to a humid atmosphere for 6 to 48 hours, preferably 12 to 36 hours, more preferably 12 to 24 hours.

In step c) the scaffold may be placed in an autoclave at a relative humidity in the range of 70%-100%, preferably in the range of 90-100%. The scaffold may be autoclaved at a temperature at or above 100 °C and a pressure at or above 1 atm of absolute pressure. Preferably the scaffold is autoclaved at a temperature above 100 °C and a pressure above 1 atm of absolute pressure. The scaffold may be autoclaved for 5 to 40 minutes, preferably 5 to 30 minutes, most preferably 5 to 10 minutes.

The hardening step d) is preferably performed by immersing the scaffold in water or an aqueous solution and autoclaving at a temperature above 100 °C and a pressure above 1 atm of absolute pressure.

The hardening step d) is preferably performed in 120 minutes or less.

The method may comprise an additional step after hardening step d). This additional step is a washing step which may be performed by immersing the hardened scaffold in water. The additional step has the effect of further removing binder from the scaffold. It also has the effect of removing the salts not incorporated in the scaffold if the scaffold was immersed in an aqueous solution.

**Figures**

Figure 1 is a graphical description of the manufacturing process.

Figure 2 shows the following images: a) optical microscopy images of the scaffolds' structure printed with orthogonal structure with 250 $\mu$m gaps within strands; b) images of the scaffolds printed at different fill densities with an orthogonal structure and 311 $\mu$m diameter strands; c) images of the scaffolds printed at different fill densities with a honeycomb structure and 311 $\mu$m diameter strands.

Figure 3 a) shows scaffolds that are 12 mm high and 6 mm wide with different patterns (260 $\mu$m strand and 45% infill)

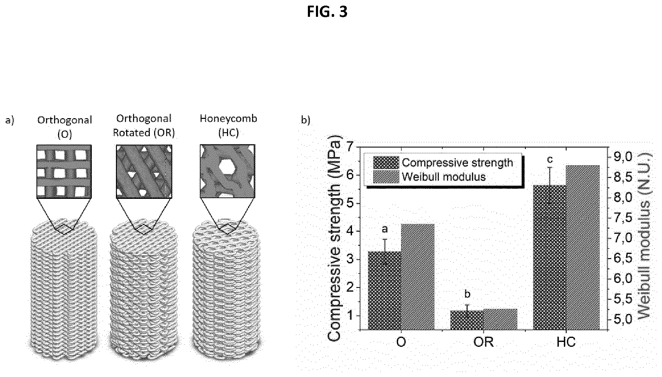

Figure 3 b) shows compressive uniaxial strength and Weibull modulus of the scaffolds printed with different patterns and subjected to the biomimetic post-printing treatment as determined by monotonic compressive loading in a universal mechanical testing machine. Different letters identify statistically significant differences in the compressive strength between treatments (One-way ANOVA with post-hoc Tukey HSD test IC=0.95).

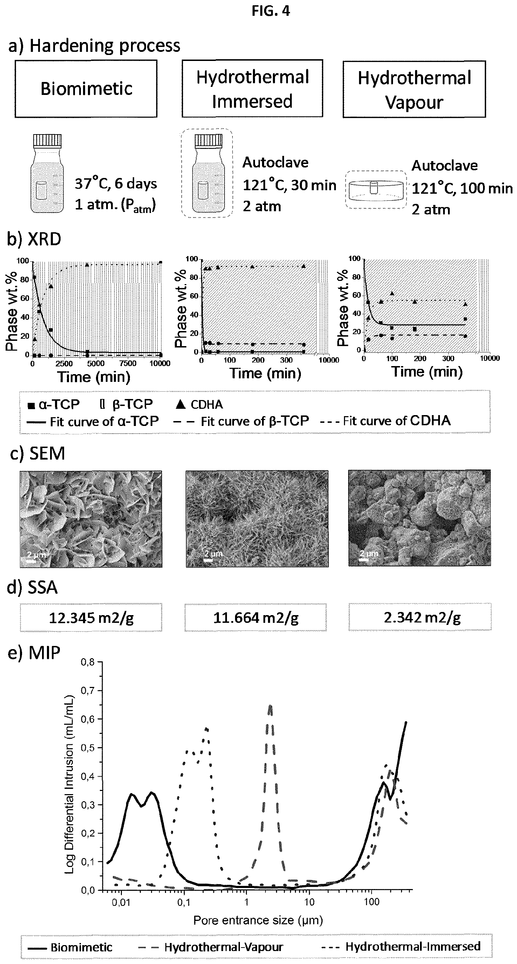

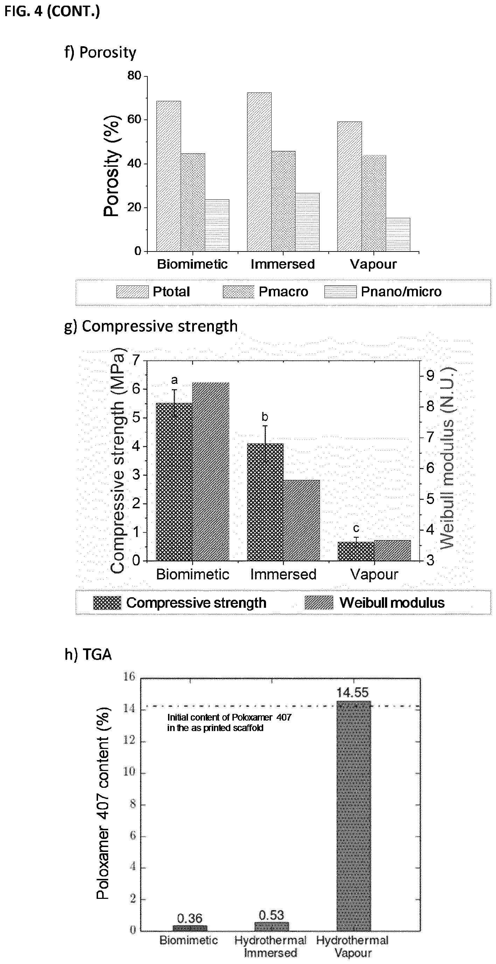

Figure 4 shows the properties of the scaffolds obtained when subjected to different hardening processes where a) shows a schematic representation depicting the 3 hardening processes; b) shows the phase transformation kinetics determined by X-ray powder diffraction (XRD) analysis for the different hardening processes; c) shows scanning electron microscopy of the microstructure of the hardened scaffold after each hardening process; d) shows specific surface area of the scaffolds for each hardening process as determined by nitrogen adsorption using the Brunauer-Emmett-Teller (BET) method; e) shows the pore entrance size distribution obtained by mercury intrusion porosimetry (MIP) for scaffolds with honeycomb pattern subjected to each of the hardening processes; f) shows the porosity of the scaffolds printed with honeycomb pattern and subjected to the different hardening processes; g) shows the compressive strength and Weibull modulus of the scaffolds set subjected to the different hardening processes as determined by monotonic compressive loading in a universal mechanical testing machine. Different letters identify statistically significant differences in the compressive strength between treatments (One-way ANOVA with post-hoc Tukey HSD test IC=0.95); h) shows the amount of poloxamer 407 in the scaffolds subjected to the different hardening processes (and not washed) calculated from the thermogravimetric analysis. The dashed line indicates the nominal concentration of poloxamer 407 in the ink.

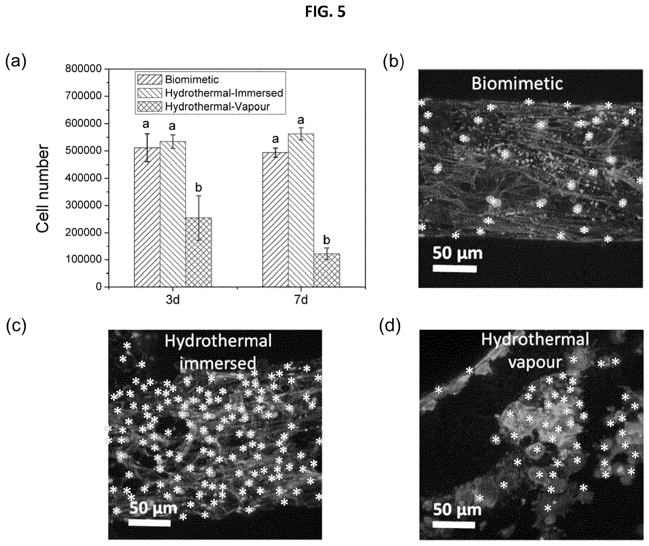

Figure 5 shows the results of the cell culture study: (a) Number of rMSC determined by LDH assay after 3 and 7 days of 3D static cell culture on orthogonal structured scaffolds subjected to the different hardening processes. Different letters identify statistically significant differences between treatments for the same time point; and (b-d) Representative confocal microscope images of cells attached to the scaffolds subjected to the different hardening processes, after 7 days of 3D static cell culture. The fibrous textures in grey correspond to the actin cytoskeleton and the nuclei are marked with asterisks (*).

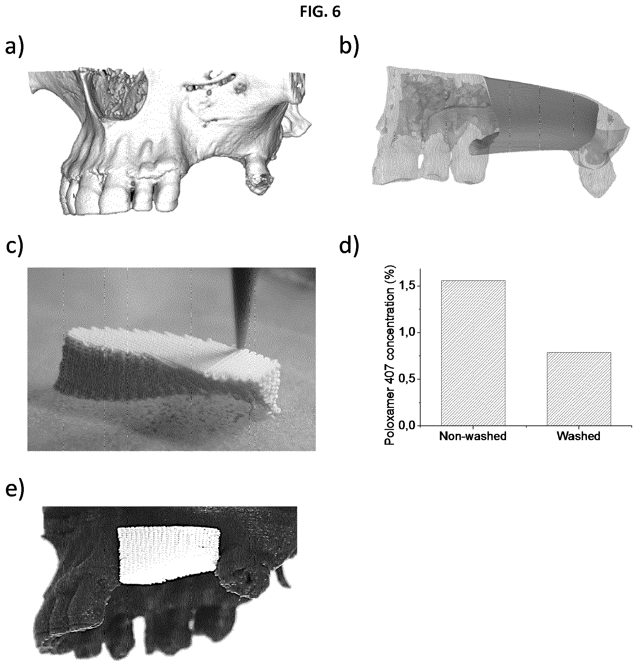

Figure 6 shows the patient-specific bone graft manufacturing process described in Example 5, where a) shows the DICOM medical image of the defect; b) shows the design of the graft based on the shape of the defect; c) shows the 3D printing process; d) shows the amount of Poloxamer 407 in the tested samples hardened under hydrothermal-immersed conditions before and after the washing step, calculated from the thermogravimetric analysis; and e) shows the fitting test on the defect printed in ABS with FDM technique.

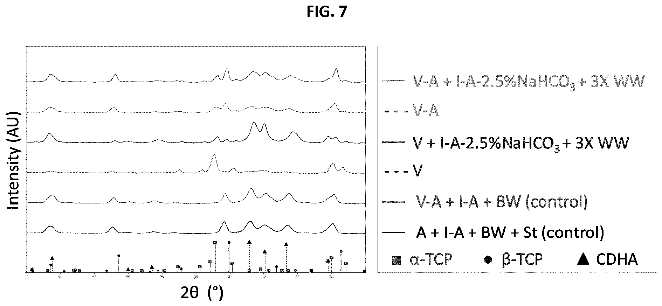

Figure 7 shows X-Ray diffraction of samples subjected to the different hardening processes described herein. Abbreviations: V:Vapour saturated bath for 15 min at 100°C,1 atm; V-A: Vapour saturated bath in autoclave for 30 min at 121°C, 2 atm; I-A: Samples immersed in distilled water and set in autoclave for 30 min at 121°C, 2 atm; I-A-2.5%NaHCO$_3$: Samples immersed in a 2.5 w./vol.% NaHCO$_3$ aqueous solution; WW: Washing of the samples in warm distilled water at 37°C for 30 min; BW: Washing of the samples in boiling distilled water for 1 hour; St: Sterilization of the samples with auto sealable sterilization bags into an autoclave for 30 min at 121°C, 2 atm.

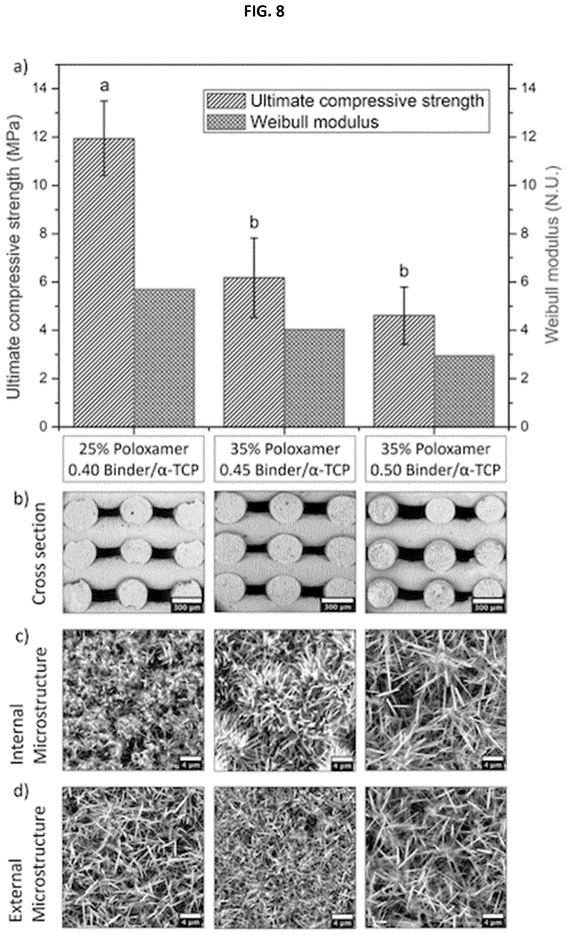

Figure 8 a) shows the uniaxial compressive strength and Weibull modulus of the scaffolds printed with the 3 different formulations presented in Table 1. Different letters identify statistically significant differences in the compressive strength between treatments (One-way ANOVA with post-hoc Tukey HSD test IC=0.95).

Figure 8 b) compares the cross-sections of the scaffolds printed with the 3 different ink formulations presented in Table 1.

Figure 8 c) compares the microstructure observed in the core of the strands of the scaffolds printed with the 3 different ink formulations presented in Table 1.

Figure 8 d) compares the microstructure observed in the surface of the strands of the scaffolds printed with the 3 different ink formulations presented in Table 1.

## Detailed description of the invention

The bone graft described herein is manufactured to perfectly fit a patient's defect. This is achieved through the use of medical imaging to obtain the shape and size of the defect, and later using the digital image to generate a personalised graft through additive manufacturing.

The composition of the graft makes it ideal for bone regeneration. Low-temperature chemical consolidation of these scaffolds makes it possible to obtain a biomimetic product, closer to bone mineral than the high-temperature sintered scaffolds. Additionally, in the low-temperature scaffolds there is no shrinkage or only very minimal shrinkage during the hardening process. During sintering, on the other hand, substantial shrinkage occurs and may be in the range of 20%. Shrinkage can lead to deviations in the final dimensions of the scaffold compared to the desired design. If a scaffold is not homogenous, shrinkage can also lead to warping of the structure.

The composition of the self-setting ink allows the transformation of $\alpha$-TCP into CDHA in a spontaneous process in which the reaction time can be controlled, as well as the nano/microstructure, composition and mechanical performance, and the ability to sustain the proliferation of mesenchymal stromal cells.

The hardening of the scaffolds is associated with the hydrolysis of the $\alpha$-TCP contained in the self-setting ink, which transforms into calcium deficient hydroxyapatite (CDHA) according to the following reaction:

$$3\text{-}Ca_3(PO_4)_2(s)+H_2O(l)\rightarrow Ca_9(HPO_4)(PO_4)_5OH\ (s)$$

When performed in an autoclave, $\beta$-TCP, a polymorph of $\alpha$-TCP may also be generated. The amount of $\beta$-TCP increases with pressure. The hardening processes that are used for the synthetic bone graft result in a synthetic bone graft with a composition comprising more than 60% of CDHA with respect to the weight of the composition. Most of the remaining weight belongs to $\beta$-TCP. A higher percentage of CDHA generally leads to better results with respect to bone regeneration.

Performing the transformation in biomimetic conditions, as described herein, yields a crystalline structure that is very similar to the mineral phase of bone, especially if carbonate is incorporated into CDHA during the hardening process. It can be accelerated with temperature and pressure, which slightly changes the structure, but preserves the high specific surface area; guaranteeing the micro and nano porosity that is necessary for adequate biological response *in vivo.*

To be used as a scaffold, the graft needs to facilitate the penetration of blood vessels throughout its volume, as well as the distribution of nutrients necessary for tissue growth. This may be achieved by controlling the porosity on a macroscopic scale, which is done in the printing process. The ink is deposited through direct injection, generating a repeating pattern, and the spaces between the extruded shapes define the size of the pore.

The pattern that is used to generate the 3-dimensional shape has a great impact on compressive strength.

The synthetic bone graft

The present invention relates to 3D-printed synthetic bone graft with a composition comprising calcium-deficient hydroxyapatite (CDHA), optionally $\alpha$-TCP and/or $\beta$-TCP.

The calcium-deficient hydroxyapatite is present in an amount of at least 60 wt.%, preferably at least 70 wt.%, preferably at least 80 wt.%, preferably at least 90 wt.% with respect to the total weight of the composition.

The present invention thus relates to a synthetic bone graft with a composition comprising calcium-deficient hydroxyapatite (CDHA) in an amount of at least 60 wt.% with respect to the total weight of the composition, optionally $\alpha$-TCP and/or $\beta$-TCP.

The composition of the synthetic bone graft further comprise a binder. The binder is present in 5 wt.% or less with respect to the total weight of the composition. The binder may be present in the range of 0.25 wt.% to 5 wt.% with respect to the total weight of the composition, preferably in the range of 0.25 wt.% to 1 wt.% with respect to the total weight of the composition.

Thus, in an embodiment the present invention relates to a synthetic bone graft with a composition comprising calcium-deficient hydroxyapatite (CDHA) in an amount of at least 60 wt.% with respect to the total weight of the composition, a binder in an amount in the range of 0.25 wt.% to 5 wt.% with respect to the total weight of the composition, optionally $\alpha$-TCP and/or $\beta$-TCP.

In another embodiment the present invention relates to a synthetic bone graft with a composition consisting of calcium phosphate in an amount of at least 95 wt.% with respect to the total weight of the composition, and a binder in an amount of 5 wt.% or less with respect to the total weight of the composition. The calcium phosphate may consist of calcium-deficient hydroxyapatite (CDHA) and optionally $\alpha$-TCP and/or $\beta$-TCP.

In a further embodiment the present invention relates to a synthetic bone graft with a composition consisting of calcium-deficient hydroxyapatite (CDHA), a binder and optionally $\alpha$-TCP and/or $\beta$-TCP. In another embodiment the present invention relates to a synthetic bone graft with a composition consisting of calcium-deficient hydroxyapatite (CDHA) in an amount of at least 60 wt.% with respect to the total weight of the composition, a binder in an amount in the range of 0.25 wt.% to 5 wt.% with respect to the total weight of the composition and optionally $\alpha$-TCP and/or $\beta$-TCP. The total amount of calcium phosphate in the composition may be at least 95 wt.% with respect to the total weight of the composition and the total amount of binder may be 5 wt.% or less with respect to the total weight of the composition.

The binder may be a poly(oxypropylene)-poly(oxyethylene) copolymer, such as poloxamer, for example Poloxamer 407 or Poloxamer P88.

The amount of $\alpha$-TCP in the composition of the synthetic bone graft may be between 0 wt.%-30 wt.%, preferably between 0 wt.%-10 wt.%, with respect to the total weight of the composition.

The amount of $\beta$-TCP in the composition of the synthetic bone graft may be between 0 wt.%-25 wt.%, preferably between 0 wt.%-15 wt.% with respect to the total weight of the composition.

The calcium-deficient hydroxyapatite in the composition of the synthetic bone graft may be doped with one or more ions. The ions may be selected from anions such as carbonate, bicarbonate, silicate, and/or cations such as Ca, Mg, Sr, Ce, Al, Zn, Ag, Co, Cu and other transition metals. Preferably the one or more ions are selected from the group consisting of carbonate, bicarbonate, silicate, calcium, strontium, zinc, cobalt and copper.

The composition of the synthetic bone graft may have a compressive strength of 2 to 12 MPa as determined by monotonic compressive loading of cylinders of 6x12 mm, preferably the compressive strength is between 4 to 12 MPa. The compressive strength may be between 2 to 6 MPa.

The composition of the synthetic bone graft may have a nano-micro porosity (a pore entrance size that is smaller than 10 micrometres) in the range of 10 % to 30%, as determined by mercury intrusion porosimetry. Preferably the composition of the synthetic bone graft has a nano-micro porosity in the range of 15 % to 25% as determined by mercury intrusion porosimetry.

The composition of the synthetic bone graft has macro-porosity (a pore entrance size that is larger than 10 micrometers) in the range of 10 % to 60% as determined by calculating the difference between the total porosity and the nano-micro porosity, wherein the nano-micro porosity is determined by mercury intrusion porosimetry. The synthetic bone graft may have macro-porosity in the range of 30 % to 50%, as determined by mercury intrusion porosimetry. Preferably the composition of the synthetic bone graft has macro-porosity in the range of 35 % to 45% as determined by mercury intrusion porosimetry. Macro-porosity may be determined by calculating the difference between the total porosity and the nano-micro porosity, according to the following formula:

$$P_{macro}(\%) = P_{TOT}(\%) - P_{micro}(\%)$$

where the nano-micro porosity is determined by mercury intrusion porosimetry. The total porosity may be determined using the following equation:

$$P_{TOT}(\%) = \left(1 - \frac{\rho_{app}\left[\frac{g}{cm^3}\right]}{\rho_{skel}\left[\frac{g}{cm^3}\right]}\right) \cdot 100$$

where skeletal density of the scaffolds (pskel) was assessed by helium pycnometry. The apparent density of the scaffolds (ρapp) was calculated as the quotient of the scaffold mass over the scaffold equivalent cylinder volume obtained from the measurements of the scaffold diameter and height.

Macroporosity can also be estimated by microcomputed tomography (micro-CT). Micro-CT uses x-rays to create cross-sections of a physical object that can be used to recreate a virtual model (3D model) without destroying the original object. The prefix micro- is used to indicate that the pixel sizes of the cross-sections are in the micrometre range.

The value measured by micro-CT corresponds to the average pore size whereas the value measured by mercury intrusion porosimetry provides the average entrance size of the macropores.

The composition of the synthetic bone graft may have a total porosity in the range of 20 % to 80%, as determined by mercury intrusion porosimetry. Preferably the composition of the synthetic bone graft has a total porosity in the range of 60 % to 80% as determined by mercury intrusion porosimetry; most preferably the total porosity is in the range of 68% to 80% as determined by mercury intrusion porosimetry. The total porosity may be determined using the following equation:

$$P_{TOT}(\%) = \left(1 - \frac{\rho_{app}\left[\frac{g}{cm^3}\right]}{\rho_{skel}\left[\frac{g}{cm^3}\right]}\right) \cdot 100$$

where skeletal density of the scaffolds ($\rho_{skel}$) was assessed by helium pycnometry. The apparent density of the scaffolds ($\rho_{app}$) was calculated as the quotient of the scaffold mass over the scaffold equivalent cylinder volume obtained from the measurements of the scaffold diameter and height.

The composition of the synthetic bone graft may have pores having diameters of less than 1 $\mu$m, as determined by mercury intrusion porosimetry.

The composition of the synthetic bone graft may have a density in the range of 2.65 to 3.14 g/cm$^3$, preferably in the range of 2.70 to 3.14 g/cm$^3$, as determined by helium pycnometry. The composition of the synthetic bone graft may have a density in the range of 2.75 to 3.14 g/cm$^3$, as determined by helium pycnometry.

The composition of the synthetic bone graft may have specific surface area (SSA) in the range of 5 to 20 m$^2$/g, as determined by nitrogen adsorption and BET analysis. Preferably the composition of the synthetic bone graft has a specific surface area (SSA) in the range of 10 to 15 m$^2$/g, as determined by nitrogen adsorption and BET analysis.

The composition of the synthetic bone graft may show a needle-like or a plate-like morphology as determined by Field Emission-Scanning Electron Microscopy. The composition of the synthetic bone graft preferably shows a needle-like morphology as determined by Field Emission-Scanning Electron Microscopy.

In another embodiment, the present invention relates to a synthetic bone graft with a composition comprising calcium-deficient hydroxyapatite (CDHA) in an amount of at least 60 wt.% with respect to the total weight of the composition, optionally $\alpha$-TCP and/or $\beta$-TCP, wherein the synthetic bone graft has nano-micro porosity in the range of 10 % to 30%.

In a further embodiment, the present invention relates to a synthetic bone graft with a composition comprising calcium-deficient hydroxyapatite (CDHA) in an amount of at least 60 wt.% with respect to the total weight of the composition, optionally $\alpha$-TCP and/or $\beta$-TCP, wherein the synthetic bone graft has macro-porosity in the range of 10 % to 60%.

In a further embodiment, the present invention relates to a synthetic bone graft with a composition comprising calcium-deficient hydroxyapatite (CDHA) in an amount of at least 60 wt.% with respect to the total weight of the composition, optionally α-TCP and/or β-TCP, wherein the synthetic bone graft has macro-porosity in the range of 30 % to 50%.

In yet a further embodiment, the present invention relates to a synthetic bone graft with a composition comprising calcium-deficient hydroxyapatite (CDHA) in an amount of at least 60 wt.% with respect to the total weight of the composition, optionally α-TCP and/or β-TCP, wherein the synthetic bone graft has a total porosity in the range of 68% to 80%.

In another embodiment, the present invention relates to a synthetic bone graft with a composition comprising calcium-deficient hydroxyapatite (CDHA) in an amount of at least 60 wt.% with respect to the total weight of the composition, optionally α-TCP and/or β-TCP, wherein the synthetic bone graft has a specific surface area (SSA) in the range of 10 to 15 $m^2$/g.

In another embodiment, the present invention relates to a synthetic bone graft with a composition consisting of calcium phosphate in an amount of at least 95 wt.% with respect to the total weight of the composition, and a binder in an amount of 5 wt.% or less with respect to the total weight of the composition, wherein the composition has a macro-porosity in the range of 10 % to 60%, as determined by calculating the difference between the total porosity and the nano-micro porosity, wherein the nano-micro porosity is determined by mercury intrusion porosimetry. The calcium phosphate may consist of calcium-deficient hydroxyapatite (CDHA) and optionally α-TCP and/or β-TCP.

In another embodiment, the present invention relates to a synthetic bone graft with a composition consisting of calcium-deficient hydroxyapatite (CDHA) in an amount of at least 60 wt.% with respect to the total weight of the composition, a binder in an amount in the range of 0.25 wt.% to 5 wt.% with respect to the total weight of the composition and optionally α-TCP and/or β-TCP, wherein the composition has a macro-porosity in the range of 10 % to 60%, as determined by calculating the difference between the total porosity and the nano-micro porosity, wherein the nano-micro porosity is determined by mercury intrusion porosimetry. The total amount of calcium phosphate in the composition may be at least 95 wt.% with respect to the total weight of the composition and the total amount of binder may be 5 wt.% or less with respect to the total weight of the composition.

## The self-setting ink

The present invention also relates to the use of a self-setting ink for the manufacture of synthetic bone graft in the form of a 3D-printed scaffold, wherein the self-setting ink comprises α-TCP powder and a binder solution as defined in claim 9.

The binder solution preferably comprises a poly(oxypropylene)-poly(oxyethylene) copolymer and water. The poly(oxypropylene)-poly(oxyethylene) copolymer may be a poloxamer, for example Poloxamer 407 or Poloxamer P88, preferably Poloxamer 407. Poloxamer 407 has a molecular mass of 4,000 g/mol and a 70% polyoxyethylene content.

Preferably the self-setting ink (essentially) consists of α-TCP powder and a binder solution made of a poly(oxypropylene)-poly(oxyethylene) copolymer (such as a poloxamer) and water. The binder solution in the self-setting ink contains 25 to 60 g poly(oxypropylene)-poly(oxyethylene) copolymer per 100 ml solution, preferably 25 to 40 g poly(oxypropylene)-poly(oxyethylene) copolymer per 100 ml solution, preferably 25 to 35 g poly(oxypropylene)-poly(oxyethylene) copolymer per 100 ml solution, for example 30 g poly(oxypropylene)-poly(oxyethylene) copolymer per 100 ml solution.

The binder solution in the self-setting ink preferably contains 25 g poloxamer per 100 ml solution.

When the binder solution in the self-setting ink contains 25 g poloxamer per 100 ml solution, the synthetic bone graft was found to have improved mechanical properties.

The binder solution in the self-setting ink contains between 25 to 60 g poloxamer per 100 ml solution. The binder solution in the self-setting ink may contain more than 30 to 60 g poloxamer per 100 ml solution, preferably 35 to 60 g poloxamer per 100 ml solution. The binder solution in the self-setting ink may contain 30 to 40 g poloxamer per 100 ml solution, preferably 30g to 35 g poloxamer per 100 ml solution. The binder solution in the self-setting ink may contain from 25 to less than 30 g poloxamer per 100 ml solution or more than 30 to 60 g poloxamer per 100 ml solution.

The binder solution in the self-setting ink may contain from 32 to 60 g poloxamer per 100 ml solution. The binder solution in the self-setting ink may contain from 25 to 28g poloxamer per 100 ml solution or from 32 to 60 g poloxamer per 100 ml solution. The binder solution in the self-setting ink may contain from 35 to 60 g poloxamer per 100 ml solution. The binder solution in the self-setting ink may contain from 25 to 60 g poloxamer per 100 ml solution, with the proviso that the binder solution in the self-setting ink does not contain 30 g poloxamer per 100 ml. The binder solution in the self-setting ink may contain from 25 to 50 g poloxamer per 100 ml solution, with the proviso that the binder solution in the self-setting ink does not contain 30 g poloxamer per 100 ml. The binder solution in the self-setting ink may contain from 25 to 40 g poloxamer per 100 ml solution, with the proviso that the binder solution in the self-setting ink does not contain 30 g poloxamer per 100 ml. The binder solution in the self-setting ink may contain from 25 to 35 g poloxamer per 100 ml solution, with the proviso that the binder solution in the self-setting ink does not contain 30 g poloxamer per 100 ml.

The binder solution in the self-setting ink may contain 25 M- to 60g poloxamer copolymer per 100 ml solution, preferably 25 to 40g poloxamer copolymer per 100 ml solution, most preferably 25 to 35 g poloxamer per 100 ml solution, for example 30 g poloxamer per 100 ml solution.

In a particularly preferred embodiment the binder solution in the self-setting ink 25 to 35g Poloxamer 407 per 100 ml solution.

The $\alpha$-TCP powder is sieved to particle sizes below 100 micrometers, preferably to particle sizes below 40 micrometers. The liquid to powder ratio is preferably between 0.2 and 0.6.

An amount of 0.3 to 0.7 g of binder solution may be used per g of $\alpha$-TCP powder. Preferably 0.4 to 0.6 g of binder solution may be used per g of $\alpha$-TCP powder.

The self-setting ink possesses thermo sensitive properties that can be adjusted by controlling the paste temperature. This may be achieved without compromising the biocompatibility of the material. It is postulated that the inclusion of a poly(oxypropylene)-poly(oxyethylene) copolymer (such as poloxamer) in the self-setting ink imparts these thermo sensitive properties on the self-setting ink. A poly(oxypropylene)-poly(oxyethylene) copolymer component (such as poloxamer) has an inverse thermal gellification behavior, which means its viscosity increases with temperature in a certain range.

The resulting self-setting ink is stable at low temperatures (-80ºC), allowing the material to be stored. It also has a relatively low injection force at room temperature, ideally between 20 and 300 N, which allows the material to be injected during the printing process. It is also cohesive at room temperature in air.

The self-setting ink may be produced by a process comprising the following steps:

a) Preparation of the binder solution.
b) Mixing of the solid and binder solution.

The preparation of the binder solution involves dissolving 25 to 60 g of poloxamer (typically poly(oxypropylene)-poly(oxyethylene) copolymer) in 20 to 50 g of water, and then diluting the compound until the desired mass percent is achieved.

The mixing of the solid and liquid phase may require the use of high speed mixing techniques.

The method of producing a synthetic bone graft

The present invention also provides a method for producing a synthetic bone graft as defined in claim 12, comprising the following steps:

a. Providing a self-setting ink as described above under the Section "The self-setting ink", such as a self-setting ink comprising $\alpha$-TCP powder and a binder solution comprising a poly(oxypropylene)-poly(oxyethylene) copolymer (such as a poloxamer) and water;
b. 3D printing to provide a scaffold;
c. placing the scaffold in a humid atmosphere; and
d. Hardening the scaffold by immersion in water or an aqueous solution consisting of water and one or more ions selected from the group consisting of anions such as carbonate, bicarbonate, silicate, and/or cations such as Ca, Mg, Sr, Ce, Al, Zn, Ag, Co, Cu and other transition metals.

*Step b):*

3D printing step b) generally involves the configuration of the printer software (including the design of the graft from medical images) followed by the preparation of the printer, the preparation of the external material (including the ink, and the means by which it is placed in the printer injection system), and the printing process itself.

The printing process is generally performed by deposition of the ink in a defined pattern. The pattern fills the contour of the shape of a slice of the graft, generating a layer, and the superposition of those layers creates the three-dimensional shape. The shape may be pre-determined by a digitalised medical imaging technique and/or computer aided design.

*Step c):*

In step c) the scaffold is placed in a humid atmosphere. Step c) is also referred to as the "pre-setting step".

In one embodiment the relative humidity of the humid atmosphere may be in the range of 70%-100%, in the range of 80%-100%, in the range of 90-100%. Preferably the relative humidity is in the range of 90-100%, and most preferably the relative humidity is 100%. The scaffold may be heated at a temperature in the range of 30 to 100°C, preferably 50 to 100°C. The scaffold may be heated at a temperature in the range of 30-50 °C, preferably 30-40 °C. The scaffold may be exposed to the humid atmosphere for 6 hours to 48 hours, preferably 12 hours to 36 hours, more preferably 12 hours to 24 hours. Such an additional step increases the cohesion of the scaffold before it is immersed in water.

In the present application, 1 atm is equivalent to 0.1 MPa.

In another embodiment the scaffold is autoclaved at a relative humidity in the range of 70%-100%, preferably in the range of 90-100%. The scaffold may be autoclaved at a pressure at or above 1 atm of absolute pressure, for example at a pressure in the range of 1 atm to 4 atm, at a pressure in the range of 1 atm to 3 atm, at a pressure in the range of 1 atm to 2 atm, at a pressure of 2 atm. The scaffold may be autoclaved at a temperature at or above 100 °C, for example at a temperature in the range of 100 to 170 °C, at a temperature in the range of 100 to 150 °C, at a temperature in the range of 100 to 130°C, at a temperature in the range of 110 to 130 °C, at a temperature in the range of 115 to 125 °C.

The scaffold may be autoclaved at a temperature at or above 100 °C and at a pressure at or above 1 atm, for example at a temperature in the range of 100 to 170 °C and at a pressure in the range of 1 atm to 4 atm, at a temperature in the range of 100 to 150 °C and at a pressure in the range of 1 atm to 4 atm, at a temperature in the range of 100 to 130 °C and at a pressure in the range of 1 atm to 4 atm, at a temperature in the range of 110 to 130 °C and at a pressure in the range of 1 atm to 4 atm, at a temperature in the range of 115 to 125 °C and at a pressure in the range of 1 atm to 4 atm, at a temperature in the range of 100 to 170 °C and at a pressure in the range of 1 atm to 3 atm, at a temperature in the range of 100 to 150 °C and at a pressure in the range of 1 atm to 3 atm, at a temperature in the range of 100 to 130 °C and at a pressure in the range of 1 atm to 3 atm, at a temperature in the range of 110 to 130 °C and at a pressure in the range of 1 atm to 3 atm, at a temperature in the range of 115 to 125 °C and at a pressure in the range of 1 atm to 3 atm at a temperature in the range of 100 to 170 °C and at a pressure in the range of 1 atm to 2 atm, at a temperature in the range of 100 to 150 °C and at a pressure in the range of 1 atm to 2 atm, at a temperature in the range of 100 to 130 °C and at a pressure in the range of 1 atm to 2 atm, at a temperature in the range of 110 to 130 °C and at a pressure in the range of 1 atm to 2 atm, at a temperature in the range of 115 to 125 °C and at a pressure in the range of 1 atm to 2 atm.

The scaffold is preferably autoclaved at a temperature above 100 °C and a pressure above 1 atm of absolute pressure. Most preferably at a temperature in the range of 115 to 125 °C and at a pressure in the range of 1 atm to 2 atm.

The scaffold may be autoclaved for 5 to 40 minutes, preferably 5 to 30 minutes, most preferably 5 to 10 minutes.

*Step d):*

In step d) the scaffold is hardened by immersion in water or an aqueous solution. The aqueous solution may contain ions that can be incorporated into the calcium-deficient hydroxyapatite (CDHA). Thus the ions may be incorporated into the scaffold while the scaffold is hardening.

The aqueous solution consists of water and one or more ions.

The ions in the aqueous solution are selected from anions such as carbonate, bicarbonate, silicate, and/or cations such as Ca, Mg, Sr, Ce, Al, Zn, Ag, Co, Cu and other transition metals. Preferably the one or more ions are selected from the group consisting of carbonate, bicarbonate, silicate, calcium, strontium, zinc, cobalt and copper.

The hardening step d) may be performed by immersing the scaffold in water or an aqueous solution at a temperature of 0 to 80°C, for example at a temperature of 0 to 30°C.

The hardening step d) may be performed by immersing the scaffold in water and heating at a temperature in the range of 30 to 80 °C, for example, at a temperature in the range of 30 to 60 °C, for example at a temperature in the range of 30 to 40 °C, such as 37 °C. The hardening step may be performed in 7 to 10 days.

The hardening step d) may be performed by immersing the scaffold in an aqueous solution consisting of water and one or more ions and heating at a temperature in the range of 30 to 80 °C, for example, at a temperature in the range of 30 to 60 °C, for example at a temperature in the range of 30 to 40 °C, such as 37 °C. The hardening step may be performed in 7 to 10 days.

The hardening step may be performed by immersing the scaffold in water and autoclaving at a temperature at or above 100 °C, for example at a temperature in the range of 100 to 170 °C, at a temperature in the range of 100 to 150 °C, at a temperature in the range of 100 to 130 °C, at a temperature in the range of 110 to 130 °C, at a temperature in the range of 115 to 125 °C.

The hardening step may be performed by immersing the scaffold in an aqueous solution consisting of water and one or more ions and autoclaving at a temperature at or above 100 °C, for example at a temperature in the range of 100 to 170 °C, at a temperature in the range of 100 to 150 °C, at a temperature in the range of 100 to 130 °C, at a temperature in the range of 110 to 130 °C, at a temperature in the range of 115 to 125 °C.

The hardening step may be performed by immersing the scaffold in water and autoclaving at a pressure at or above 1 atm of absolute pressure, for example at a pressure in the range of 1 atm to 4 atm, at a pressure in the range of 1 atm to 3 atm, at a pressure in the range of 1 atm to 2 atm, at a pressure of 2 atm.

The hardening step may be performed by immersing the scaffold in an aqueous solution consisting of water and one or more ions and autoclaving at a pressure at or above 1 atm of absolute pressure, for example at a pressure in the range of 1 atm to 4 atm, at a pressure in the range of 1 atm to 3 atm, at a pressure in the range of 1 atm to 2 atm, at a pressure of 2 atm.

The hardening step may be performed by immersing the scaffold in water and autoclaving at a temperature at or above 100 °C and at a pressure at or above 1 atm, for example at a temperature in the range of 100 to 170 °C and at a pressure in the range of 1 atm to 4 atm, at a temperature in the range of 100 to 150 °C and at a pressure in the range of 1 atm to 4 atm, at a temperature in the range of 100 to 130 °C and at a pressure in the range of 1 atm to 4 atm, at a temperature in the range of 110 to 130 °C and at a pressure in the range of 1 atm to 4 atm, at a temperature in the range of 115 to 125 °C and at a pressure in the range of 1 atm to 4 atm, at a temperature in the range of 100 to 170 °C and at a pressure in the range of 1 atm to 3 atm, at a temperature in the range of 100 to 150 °C and at a pressure in the range of 1 atm to 3 atm, at a temperature in the range of 100 to 130 °C and at a pressure in the range of 1 atm to 3 atm, at a temperature in the range of 110 to 130 °C and at a pressure in the range of 1 atm to 3 atm, at a temperature in the range of 115 to 125 °C and at a pressure in the range of 1 atm to 3 atm, at a temperature in the range of 100 to 170 °C and at a pressure in the range of 1 atm to 2 atm, at a temperature in the range of 100 to 150 °C and at a pressure in the range of 1 atm to 2 atm, at a temperature in the range of 100 to 130 °C and at a pressure in the range of 1 atm to 2 atm, at a temperature in the range of 110 to 130 °C and at a pressure in the range of 1 atm to 2 atm, at a temperature in the range of 115 to 125 °C and at a pressure in the range of 1 atm to 2 atm.

The hardening step may be performed by immersing the scaffold in an aqueous solution consisting of water and one or more ions and autoclaving at a temperature at or above 100 °C and at a pressure at or above 1 atm, for example at a temperature in the range of 100 to 170 °C and at a pressure in the range of 1 atm to 4 atm, at a temperature in the range of 100 to 150 °C and at a pressure in the range of 1 atm to 4 atm, at a temperature in the range of 100 to 130 °C and at a pressure in the range of 1 atm to 4 atm, at a temperature in the range of 110 to 130 °C and at a pressure in the range of 1 atm to 4 atm, at a temperature in the range of 115 to 125 °C and at a pressure in the range of 1 atm to 4 atm, at a temperature in the range of 100 to 170 °C and at a pressure in the range of 1 atm to 3 atm, at a temperature in the range of 100 to 150 °C and at a pressure in the range of 1 atm to 3 atm, at a temperature in the range of 100 to 130 °C and at a pressure in the range of 1 atm to 3 atm, at a temperature in the range of 110 to 130 °C and at a pressure in the range of 1 atm to 3 atm, at a temperature in the range of 115 to 125 °C and at a pressure in the range of 1 atm to 3 atm, at a temperature in the range of 100 to 170 °C and at a pressure in the range of 1 atm to 2 atm, at a temperature in the range of 100 to 150 °C and at a pressure in the range of 1 atm to 2 atm, at a temperature in the range of 100 to 130 °C and at a pressure in the range of 1 atm to 2 atm, at a temperature in the range of 110 to 130 °C and at a pressure in the range of 1 atm to 2 atm, at a temperature in the range of 115 to 125 °C and at a pressure in the range of 1 atm to 2 atm.

The hardening step d) is preferably performed by immersing the scaffold in water or an aqueous solution consisting of water and one or more ions and autoclaving at a temperature above 100 °C and a pressure above 1 atm of absolute pressure.

The hardening step d) is preferably performed in 120 minutes or less, for example the hardening step d) is performed in 5 to 120 minutes, in 10 to 120 minutes, in 20 to 120 minutes, in 5 to 60 minutes, in 10 to 60 minutes or in 20 to 40 minutes.

The hardening step d) is preferably performed until the binder content of the scaffold has dropped to 5 wt.% or less with respect to the total weight of the scaffold, for example until the binder content is within a range of 0.25 wt.% to 5 wt.%, preferably in the range of 0.25 wt.% to 1 wt.% with respect to the total weight of the scaffold.

The binder may be a poly(oxypropylene)-poly(oxyethylene) copolymer, such as poloxamer, for example, Poloxamer 407 or Poloxamer P88, preferably Poloxamer 407.

*Optional additional step:*

The method may comprise an additional step after hardening step d). This additional step is a washing step which may be performed by immersing the hardened scaffold in water. The additional step has the effect of further removing binder from the scaffold. This is shown in Figure 6 e) where a decrease in poloxamer concentration in the scaffold was observed after the washing step.

In one embodiment, the washing step comprises immersing the scaffold in water, preferably distilled water, having a temperature above 80°C, preferably having a temperature in the range of 90°C to 100°C. The sample may be immersed in water for 30 minutes to 2 hours, preferably for 45 minutes to 1.5 hours. Preferably the sample is immersed in water having a temperature in the range of 90°C to 100°C for 45 minutes to 1.5 hours. To increase the effectiveness of the washing step, the water can be stirred with an agitator.

In another embodiment, the washing step comprises immersing the scaffold in water, preferably distilled water, having a temperature in the range of 1 to 10 °C, preferably having a temperature in the range of 2°C to 5°C. The sample may be immersed in water for 24 hours to 60 hours, preferably for 36 hours to 48 hours. Preferably the sample is immersed in water having a temperature in the range of 2°C to 5°C for 36 hours to 48 hours. To increase the effectiveness of the washing step, the water can be stirred with an agitator.

When Poloxamer 407 is used as the binder in the self-setting ink then it is thought that the additional washing step enhances the release of Poloxamer 407 from the scaffold because the temperatures the scaffolds are exposed to in each of the washing step embodiments are outside the gellification range of Poloxamer 407 solutions.

*Optional combined hardening and washing step:*

In one embodiment, hardening step d) and the optional washing step may be carried out simultaneously. This may be achieved by periodically changing the immersing solution during hardening step d). For example, the hardening step may be carried out for a certain period of time, stopped, the immersing solution changed and the hardening step started again. This process can be repeated a number of times and will have the effect of washing the scaffold during the hardening step.

In one embodiment, the immersing solution can be changed 2 to 5 times during hardening step d), preferably 2 to 3 times. The immersing solution can be changed at regular intervals or irregular intervals.

The immersing solution may be water or an aqueous solution. The aqueous solution employed may contain one or more ions. These ions may be selected from anions such as carbonate, bicarbonate, silicate, and/or cations such as Ca, Mg, Sr, Ce, Al, Zn, Ag, Co, Cu, and other transition metals.

Preferably the one or more ions are selected from the group consisting of carbonate, bicarbonate, silicate, calcium, strontium, zinc, cobalt and copper.

Applications

The composition comprising calcium-deficient hydroxyapatite (CDHA) described herein is biomimetic and structurally (on a macro and micro scale) similar to the mineral phase of bone. This makes it a particularly suitable material for bone grafting and thus for use as a synthetic bone graft.

The synthetic bone graft described herein may be used in the following locations in a body:

- cranio-maxillo facial / dental: sinus lift, alveolar filling, peri-implantation filling, affixed graft,
- inlay/ onlay graft, maxillary distraction. cranio-facial plastic. orbital floor
- spine: spine fusion, spinal cage filling
- Iliac crest harvesting
- upper limb: humeral head fracture, glenoidal prosthesis, humeral fracture, distal radius
- fracture
- lower limb: femoral head fracture, femoral nail revision, screws ablation, hip prosthesis,
- knee prosthesis, tumoral filling, tibial fracture
- foot: calcaneum fracture, phalanx fusion, talus fusion.

Examples

The invention is illustrated with the following non-limiting examples.

Example 1: Preparation of a self-setting ink

A binder solution containing 35 g poloxamer 407 per 100 ml solution was prepared. The dissolution was obtained by mixing the 35g of Poloxamer 407 with 35 mL of distilled water with a dual asymmetric centrifugal laboratory mixer for 5 minutes at 2350 rpm and subsequently adding the rest of the water prior to mixing for 10 more minutes at 2350 rpm. 6 g of alpha-tricalcium phosphate ($d_{10}$=1.03 $\mu$m, $d_{50}$=4.08 $\mu$m, $d_{90}$=12.13 $\mu$m) were mixed with 3 g of the 30 wt./vol.% Poloxamer 407 aqueous solution. The mixing was carried out in a dual asymmetric centrifugal laboratory mixer during 30 seconds at 3500 rpm.

Example 2: 3D printing - control of the macrostructure, scaffold structure (pattern) and pore size.

The paste obtained in Example 1 was placed into a 3cc capacity BD Luer-Lok™ syringe barrel through the bottom of the syringe with the help of a spatula. Then a plastic tapered dispense tip was coupled to the barrel. Three different tip Birmingham gauges were used in order to control the strand diameter: Gauge 22, 24 and 25; obtaining strands of 413 $\mu$m, 311 $\mu$m, and 260 $\mu$m respectively.

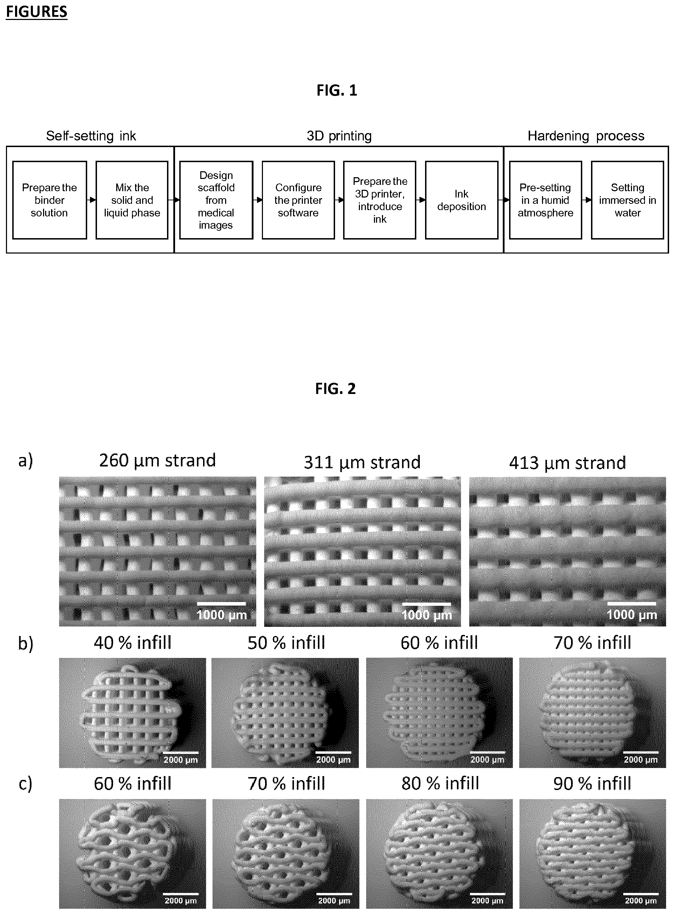

Figure 2a) shows optical microscopy images of scaffolds with strands printed with tapered tips of different inner diameter. Images were acquired with an optical microscope (SZX16, Olympus, Japan) equipped with a digital camera sensor (uEye, IDS Imaging Development Systems GmbH).

Simultaneously, cylinders that were 12mm high and 6 mm wide were designed with a computer-aided design (CAD) software. Parts were exported to a mesh format (. STL) and imported in a Slicing software where the printer pre-sets such as layer height, layer width, nozzle diameter, nozzle speed, printing pattern (internal scaffold structure) and percentage of infill (degree of porosity) were defined. Different patterns, nozzle diameters, and infill percentages were compared.

Figure 2b) shows optical microscopy images of scaffolds with different infill percentage. This variable is directly correlated to the scaffolds pore size. Images were acquired with an optical microscope (SZX16, Olympus, Japan) equipped with a digital camera sensor (uEye, IDS Imaging Development Systems GmbH).

Figure 3 shows the ultimate compressive strength of scaffolds with different strand patterns: Orthogonal (O), Orthogonal Rotated (OR) and Honeycomb (HC); All the samples in this test were hardened under the biomimetic setting treatment. The ultimate compressive strength was determined by monotonic compressive loading of cylindrical scaffolds with a diameter of 6 mm and a height of 12 mm (6x12 mm) printed with different internal structures. A universal testing machine (Bionix, MTS) was employed to perform the uniaxial compressive tests at a rate of 1 mm min$^{-1}$. The load was applied perpendicular to the printing plane (XY plane), i.e. in the Z direction. Samples were tested in a wet state in order to simulate an *in vivo* environment. A total of 12 scaffolds were tested for each condition. The Weibull modulus was calculated applying a Weibull statistical analysis on the ultimate compressive stress values (Eq. 1).

$$Ln\left(Ln\left(\frac{1}{P_s}\right)\right) = m(Ln(\sigma) - Ln(\sigma_0)) \tag{1}$$

Where:

    Ps = Probability of survival
    $\sigma$ = ultimate compressive strength of the sample
    $\sigma$0 = constant corresponding to the stress for which 100 % of the samples will not fail
    m = Weibull modulus

Then, all the information of the printing coordinates of the motors for the printer was exported in a C-programing language file (G-code).

The syringe assembly was installed in a 3D paste microextruder printer. The printer used was a custom designed machine adapted from a commercial fused deposition modeling printer (BCN3D+, BCN3D Technologies, Spain). It mainly consists of a 3 cartesian axis system (X, Y, Z) that controls the position of a carriage where a microextruder is assembled. The microextruder is based on a stepper motor coupled to a piston that controls the displacement of the syringe plunger.

Once the syringe was placed, the G-code was sent to the machine to be executed and the paste was deposited layer by layer creating a 3D structure.

Example 3 - Pre-setting step

Once the process finished the result was a green scaffold composed of an unreacted alpha-tricalcium phosphate ($\alpha$-TCP) powder bound together by the poloxamer 407 gel described hereinbefore. The pre-setting step consisted in applying a 100% relative humidity bath to the scaffold at 37°C for 24 hours. During the pre-setting step a small part of the $\alpha$-TCP was transformed by hydrolysis to calcium-deficient hydroxyapatite (CDHA).

Following the pre-setting step, the scaffold was immersed in 37°C distillate water for 6-days to achieve the transformation of the $\alpha$-TCP into CDHA.

Example 4 - Control of the microstructure through different hardening processes.

The green scaffolds obtained in Example 2 were subjected to three different hardening treatments.

The first treatment involved in a pre-setting step of 24 hours in a water-saturated atmosphere at 37°C and 1 atm of absolute pressure. The was followed by a hardening step referred to as a "Biomimetic Treatment" involving immersion of the scaffolds in a water bath at 37°C for 6 days.

The second treatment involved a pre-setting step of 10 minutes in a vapour saturated atmosphere at 100°C and 1 atm of absolute pressure. This was followed by a hardening step referred to as a "Hydrothermal-Immersed" treatment wherein the scaffolds were immersed in water and autoclaved in a wet cycle (steam) at 121°C and 2 atm of absolute pressure for 30 minutes.

The third treatment involved a pre-setting step of 10 minutes in a vapour saturated atmosphere at 100°C and 1 atm of absolute pressure. This was followed by a hardening step referred to as "Hydrothermal-Vapour" treatment that consisted in autoclaving the scaffolds in a 100 minutes cycle in a wet cycle (steam) at a temperature of 121°C and 2 atm of absolute pressure.

The influence of the post-printing treatment on cell response was studied in static 3D culture conditions in samples fabricated with O architecture. Scaffolds (discs of 6 mm in diameter and 2 mm in height) were placed in 24-well cell culture plates, sterilised with ethanol (70 %, Sigma and Aldrich) during 30 min and rinsed 3 times with phosphate buffer saline (PBS, Gibco). Samples were then preconditioned for 2 hours with 1 ml of advanced Dulbecco's Modified Eagle Medium supplemented with 10% fetal bovine serum, 2 mM L-glutamine, 50 U mL$^{-1}$ penicillin, 50 $\mu$g mL$^{-1}$ strep-tomycin and 20 mM HEPES. Afterwards, the medium was removed and $5\times10^5$ rat mesenchymal stromal cells (rMSC; extracted from bone marrow of long bones of Lewis rats; in passages 3 to 5) were seeded on each scaffold in a 25 $\mu$l medium drop. rMSC were allowed to attach during 1 h at 37 °C and 5 % CO2 and then 1 ml of fresh medium was gently added. After 3 and 7 days of static cell culture, the scaffolds were moved to a new well plate, rinsed with PBS and the remaining cells on the scaffolds were lysed with 500 $\mu$l of M-PER (mammalian protein extraction reagent, Thermo Scientific, Waltham, MA, USA). The cell number was quantified using a commercial lactate dehydrogenase (LDH) detection kit (Roche Applied Science, Penzberg, Germany). The LDH activity in the lysates was measured spectrophotometrically at a wavelength of 492 nm with PowerWave HT Microplate Reader (BioTek Instruments, Inc., Winooski, VT). A calibration curve with decreasing number of cells was used to express the results of LDH activity in cell number. Experiments were performed in triplicates for statistical analysis.

Immunofluorescent staining was used to visualise cell morphology. After 7 days of culture the scaffolds were rinsed in PBS-glycine and cells were fixed for 20 min in 4 % paraformaldehyde solution. Cells were permeabilised for 15 min with Triton X-100 (0.05 %) and blocked for 30 min in PBS-bovine serum albumin (BSA; 1 %). Actin filaments were stained with TRITC-conjugated phalloidin (Sigma-Aldrich, USA) and nuclei were counterstained with DAPI (4',6-diamidino-2-phenylindole, 1 $\mu$g/ml). Images were acquired with an E600 fluorescence microscope (Nikon, Japan) and treated with Cell F software (Olympus, Japan).

In Figure 4a) a schematical representation of each treatment and main conditions is depicted.

Figure 4b) shows how the scaffold composition can be tuned with the hardening treatment applied and how biphasic CaP scaffolds containing $\beta$-TCP and CDHA can be obtained with an hydrothermal-immersed process. Phase composition was assessed at different reaction times by X-ray powder diffraction on the powder obtained for each hardening condition after manually crushing the scaffolds in an agate mortar. In the case of scaffolds subjected to the biomimetic treatment, samples were immersed in isopropanol during 15 min prior to the analysis to stop the hardening reaction at selected times. The diffractometer (D8 Advance, Bruker) equipped with a Cu K$\alpha$ X-ray tube was operated at 40 kV and 40 mA. Data were collected in 0.02° steps over the 2$\theta$ range of 10-80° with a counting time of 2 s per step. Phase quantification was performed using the reference intensity ratio method (EVA, Bruker) comparing diffraction patterns of the crystalline structures of $\alpha$-TCP (ICDD PDF 00-029-0359), CDHA (ICDD PDF 01-086-1201) and $\beta$-TCP (ICDD PDF 00-003-0681).

Figure 4c) shows that the different hardening treatments influenced the microstructure of the scaffolds as observed by Field Emission Scanning Electron Microscopy (FIB/SEM, Zeiss Neon 40) at 5 kV, after coating the surface with a thin carbon layer. The biomimetic treatment resulted in plate-like structures whereas the hydrothermal-immersed treatment resulted in needle-like CDHA crystals. For the hydrothermal-vapour treatment much smaller crystals were formed.

Figure 4d) shows how the crystal shape influences the scaffold specific surface area (SSA) as determined by nitrogen adsorption using the BET (Brunauer-Emmett-Teller) method (ASAP 2020, Micromeritics). Prior to measurement, samples were outgassed in vacuum conditions (10 mmHg) at a holding temperature of 100 °C for 2 h.

Figure 4e) compares the open pore entrance size distribution determined mercury intrusion porosimetry (MIP, AutoPore IV Micromeritics) in the range between 0.006 and 360 $\mu$m for the different hardening processes. A multi-scale porosity profile can be observed for all the treatments: The micro-nano porosity (pore entrance size <10 $\mu$m) which presents variations in its range depending on the treatment used, as it depends on the microstructure of the material, and the macroporosity (pore entrance size > 10 $\mu$m) defined by the structure of the scaffold.

Figure 4f) compares the porosity of the scaffolds obtained with different hardening processes. In order to calculate the porosity, the volume of nano-micropores normalised per unit of mass (Vmicro) was obtained from the sum of the incremental mercury intrusion in the pores smaller than 10 $\mu$m measured in the pore entrance size distribution curves presented in the Figure 4e). The skeletal density of the scaffolds ($\rho$skel) was assessed by helium pycnometry (AccuPyc 1330, Micromeritics, USA). The apparent density of the scaffolds ($\rho$app) was calculated as the quotient of the scaffold mass over the scaffold equivalent cylinder volume obtained from the measurements of the scaffold diameter and height. All the tests were conducted with honeycomb patterned 6x12 mm cylindrical scaffolds.

The value of the total porosity (PTOT) was calculated with the Eq.2[35]:

$$P_{TOT}(\%) = \left(1 - \frac{\rho_{app}\left[\frac{g}{cm^3}\right]}{\rho_{skel}\left[\frac{g}{cm^3}\right]}\right) \cdot 100 \qquad (2)$$

The nano-microporosity (Pmicro) was calculated from mercury intrusion porosimetry data using Eq. 3:

$$P_{micro}(\%) = \left(V_{micro}\left[\frac{cm^3}{g}\right] \cdot \rho_{app}\left[\frac{g}{cm^3}\right]\right) \cdot 100 \qquad (3)$$

where ($V_{micro}$) is the volume of nano/micropores normalised per unit of mass, which was determined as the sum of the incremental mercury intrusion in the pores smaller than 10 micrometers.

The macroporosity (Pmacro) was calculated as the difference between the total porosity and the nano-micro porosity (Eq.4):

$$P_{macro}(\%) = P_{TOT}(\%) - P_{micro}(\%) \qquad (4)$$

Figure 4g) shows how the microstructure is directly correlated to the mechanical properties of the final scaffold as determined by monotonic compressive loading. A higher aspect ratio of the crystals led to a higher level of entanglement thus resulting in a higher compressive strength. The compression assays were carried out with honeycomb patterned cylindrical 6x12 mm scaffolds. The tests and subsequent analysis were conducted using exactly the same procedure described for the Figure 3.

Figure 4h) shows the amount of binder retained in the structure after the different setting treatments measured by thermogravimetric analysis. It can be seen that for the biomimetic and hydrothermal-immersed hardening treatments most of the binder was washed out during the treatment. However, no release of binder was observed for the hydrothermal-vapour treated samples. Thermogravimetric analysis (TGA) was performed under a heating rate of 10°C min-1 from 25 to 550°C in an oxygen saturated atmosphere. The amount of binder contained in the scaffolds was determined by comparing the TGA curves of each sample with those of a binder-free counterpart, consisting of $\alpha$-TCP mixed with water with the same ratio and subjected to the same treatment as the corresponding sample.

Figure 5 shows the results of an *in vitro* test performed on the scaffolds set under the different conditions. 5 million rat mesenchymal stem cells were seeded on the scaffolds. The results show good adhesion and no cytotoxicity for the biomimetic and hydrothermal-immersed treated samples, however, a low cell adhesion and proliferation for hydrothermal-vapour samples was observed.

Example 5. Patient-specific defect

A cone beam computed tomography (CBCT) of a dental defect was received together with the clinical indications of a dental surgeon. A 3D mesh was segmented based on the received Digital Imaging and Communications in Medicine (DICOM) file with the help of a medical image treatment software (Figure 6a)). Then, the mesh was imported into computer-aided design (CAD) software and a negative of the defect was obtained with Boolean operations. The external side of the graft which is not in contact with the bone was designed according to the surgeon's indications (Figure 6b)). After, this CAD design of the bone graft was exported as mesh format (.STL) and imported in a Slicing software, the printer pre-sets such as layer height, layer width, nozzle diameter, nozzle speed, printing pattern (internal scaffold structure) and percentage of infill (degree of porosity) were fixed.

6 g of alpha-tricalcium phosphate ($d_{10}$=1.03 $\mu$m, $d_{50}$=4.08 $\mu$m, $d_{90}$=12.13 $\mu$m) were mixed with 3 g of the 35 wt./vol.% Poloxamer 407 aqueous solution. The mixing was carried out in a dual asymmetric centrifugal laboratory mixer and consisted in 3 runs of the following mixing program: 30 seconds at 800 rpm followed by 1 minute at 2300 rpm and finally 30 seconds at 1150 rpm.

The paste obtained was placed into a 5CC capacity BD Luer-Lok™ syringe barrel through the back of the syringe with the help of a spatula. Then a gauge 25 (260 $\mu$m inner diameter) plastic tapered dispense tip was coupled to the barrel.

The syringe assembly was installed in a 3D paste microextruder printer and the printing process of the scaffolds took place as described in the Example 1. Figure 6c) illustrates this process.

After the printing process, a pre-setting step was applied to the green scaffold. The process consisted in applying to the graft a 30-minute autoclave cycle under wet (vapour saturated atmosphere) conditions at 121ºC and 2 atmospheres (absolute pressure).

Then a hardening step was carried out: the scaffold was immersed into a beaker filled with distillate water and placed in an autoclave. The same wet 30-minute program (at 121°C and 2 atm of absolute pressure) was applied.

After, the scaffold was washed with water so as to completely eliminate the traces of binder in the structure. This step could either be done in warm water by immersing the part into boiling water (at atmospheric pressure) for one hour or by keeping the scaffold immersed into cold water (4°C) stored inside a fridge for two days. In any case, the ratio of water per weight of scaffold was set at a minimum ratio of 15 (wt./wt.). Figure 6d) shows the comparison of the binder content in the scaffold before and after the washing step measured by TGA with exactly the same procedure described for the figure 4h).

Finally, the scaffold was packaged into a double self-sealing sterilization pouch and sterilised. The sterilization could be carried out either by autoclaving the product with a wet program (30 minutes, 121°C and 2 atm of absolute pressure) or by gamma radiation with a minimum dose of 25KGys.

In order to assess whether this technology was capable of printing complex structures and fitting into a real bone defect, a replica of the bone defect was printed in acrylonitrile butadiene styrene (ABS) with a fused deposition modeling (FDM) device. A perfect fitting of the scaffold is shown in Figure 6e).

Example 6. Ionic substitution during the hardening process

A paste composed of alpha-Tricalcium Phosphate and a Poloxamer 407 aqueous solution was obtained using the same procedure described in the Example 1. This paste was placed in a 3CC BD Luer-Lok™ syringe and used to print cylinders of 6mm height and 6mm width using the same procedure described in Example 2.

The resulting green scaffolds were pre-set with vapour by either placing them in a 100% relative humidity bath at 100 °C during 15 minutes or by applying an autoclave cycle: 121°C, 30 min, vapour, 2 atm of absolute pressure.

25 ml of sodium bicarbonate ($NaHCO_3$) were dissolved in 1000 ml of miliQ water in a glass beaker. The dissolution was carried out in agitation with the help of a magnetic stirrer at room temperature obtaining a 2.5 w./vol.% $NaHCO_3$ aqueous solution.

6 cylindrical scaffolds of 6x12 mm with O pattern were immersed in a beaker with 500 ml of $NaHCO_3$ aqueous solution at a rate of 562 ml/g of sample. The beaker was placed inside an autoclave and the following program was run: 121°C, 30 min, vapour, 2 atm of absolute pressure.

Afterwards, the scaffolds were immersed in 3 consecutive baths of 500 ml miliQ water at 37°C for 30 minutes in order to wash the samples from the carbonate ions not incorporated in the CDHA lattice.

Figure 7 shows the X-ray diffraction patterns of the scaffolds hardened with the "Hydrothermal Immersed" treatment described in Example 4 (control) compared to scaffolds hardened with a "Hydrothermal Immersed" treatment using a $NaHCO_3$ solution. The displacement of the XRD peaks indicates the incorporation of carbonate in the apatitic structure. It was observed that when the pre-setting step was conducted at atmospheric pressure and for a short time (15min) the incorporation of $CO_3$ was higher. The content of carbonate for the scaffolds processed using the $NaHCO_3$ solution was analyzed by bulk combustion using a Thermal combustion element analyzer (Thermo EA 1108). The analysis showed that the amount of carbonate introduced in the CDHA when the pre-setting was carried out at atmospheric pressures was 12%.

Example 7. Printing with different % of Poloxamer P407 in the binder phase and different proportions of binder solution per g of α-TCP powder: influence on the mechanical properties

Ink formulations with different amounts of Poloxamer P407 in the binder solution were developed. Reducing the amount of Poloxamer in the binder solution resulted in a lower viscosity, which allowed the amount of α-TCP powder incorporated in the ink to be increased. Different proportions of binder solution and powder phase were tested. The ink formulations studied are summarized in Table 1.

Two binder solutions were prepared, containing either 25 or 35 g Poloxamer 407 per 100 ml solution. The first binder solution was obtained by mixing 25 g of Poloxamer 407 with 25 ml of distilled water with a dual asymmetric centrifugal laboratory mixer for 5 minutes at 2350 rpm and subsequently adding the rest of the water prior to mixing for 10 more minutes at 2350 rpm. The second binder solution was obtained by mixing 35 g of Poloxamer 407 with 35 ml of distilled water with a dual asymmetric centrifugal laboratory mixer for 5 minutes at 2350 rpm and subsequently adding the rest of the water prior to mixing for 10 more minutes at 2350 rpm. In both cases, if the mixture was not homogeneous, the mix was cooled down to 4°C and mixed for 10 more minutes at 2350 rpm.

These two binder solutions, containing either 25 or 35% Poloxamer 407 were used to obtain three different ink formulations as summarized in Table 1.

The binder solution containing 35% Poloxamer was mixed with α-TCP powder ($d_{10}$=1.03 μm, $d_{50}$=4.08 μm, $d_{90}$=12.13 μm) at two proportions, either 0.50 g of binder solution per g of α-TCP powder (this is, 3g of binder solution were mixed with 6g of α-TCP) or 0.45 g of binder solution per g of α-TCP powder (this is, 2.7 g of binder solution were mixed with 6g of $\alpha$-TCP).

The binder solution containing 25% Poloxamer was mixed with the $\alpha$-TCP powder ($d_{10}$=1.03 $\mu$m, $d_{50}$=4.08 $\mu$m, $d_{90}$=12.13 $\mu$m) at a proportion of 0.4 g of binder solution per g of $\alpha$-TCP powder (this is, 2.4 g of binder solution were mixed with 6g of $\alpha$-TCP).

In all cases the mixing was carried out in a dual asymmetric centrifugal laboratory mixer during 30 seconds at 3500 rpm.

*Table 1. Paremeters used in the different ink formulations:*

| Concentration of Poloxamer 407 solution (wt./vol. %) | g of binder solution per g of $\alpha$-TCP powder |
|---|---|
| 35 | 0.50 |
| 35 | 0.45 |
| 25 | 0.40 |

The paste obtained was placed into a 3CC capacity BD Luer-Lok™ syringe barrel through the back of the syringe with the help of a spatula. Then a gauge 25 (260 $\mu$m inner diameter) plastic tapered dispense tip was coupled to the barrel.

12 cylindrical scaffolds with 6 mm in diameter and 12 mm in height were printed with each ink formulation. The percentage of infill was set to 55% (213 $\mu$m pores in the XY plane), the layer height was set to 200 $\mu$m and the pattern was and the pattern chosen was orthogonal (O).

The obtained green scaffolds received the same pre-setting, hardening and sterilization treatments as the ones described in Example 5.

Figure 8 a) shows the ultimate compressive strength and the Weibull modulus of the scaffolds printed with the three different ink formulations presented in Table 1. The ultimate compressive strength was determined by monotonic compressive loading of cylindrical scaffolds with a diameter of 6 mm and a height of 12 mm (6x12 mm) with a universal testing machine (Bionix, MTS) applying the same procedure described in the Example 2 (Figure 3). Reducing the amount of Poloxamer in the binder solution allowed incorporating a higher proportion of $\alpha$-TCP powder in the inks. This is relevant because a higher amount of ceramic content in the ink (lower binder to $\alpha$-TCP ratio) led to scaffolds with higher compressive strength and more homogeneously distributed defects in the material (resulting in higher Weibull modulus). The formulation with 25% Poloxamer and 0.4 g of binder per g of $\alpha$-TCP powder had double the compressive strength compared to the formulation with 35% Poloxamer and 0.45 g of binder per g of $\alpha$-TCP powder. The formulation with 25% Poloxamer and 0.4 g of binder per g of $\alpha$-TCP powder had nearly triple the compressive strength compared to the formulation with 35% Poloxamer and 0.50 g of binder per g of $\alpha$-TCP powder.

Figure 8 b) shows no significant differences in the cross-sections of the scaffolds printed with the 3 different ink formulations described in Table 1.

Figure 8 c) shows differences in the inner microstructure of the scaffolds printed with the 3 different ink formulations described in Table 1. The length of the crystals is reduced with lower L/P ratios.

Figure 8 d) presents the microstructure of the material at the surface of the strands of the scaffolds printed with the 3 different ink formulations described in Table 1.

Images in Figures 8 b), c) and d) were acquired by scanning electron microscopy (Phenom XL, Phenom-World B.V., ThermoFisher Scientific, Eindhoven, The Netherlands) with a backscattered electron detector (BSE) at an intensity of 10kV. Prior to analysis a thin carbon coating was deposited on the samples.

## References

[1] C. G. Finkemeier, J. Bone Joint Surg. Am. 2002, 84-A, 454.

[2] M. Bohner, Mater. Today 2010, 13, 24.

[3] B. Stevens, Y. Yang, A. Mohandas, B. Stucker, K. T. Nguyen, J. Biomed. Mater. Res. Part 8 Appl. Biomater. 2008, 858, 573.

[4] S. Bose, S. Vahabzadeh, A. Bandyopadhyay, Mater. Today 2013, 16, 496.

[5] D. Tang, R. S. Tare, L. Y. Yang, D. F. Williams, K. L. Ou, R. O. C. Oreffo, Biomaterials 2016, 83, 363.

[6] A. Butscher, M. Bohner, S. Hofmann, L. Gauckler, R. Müller, Acta Biomater. 2011, 7, 907.

[7] J. Cesarano, T. A. Baer, P. Calvert, in Proc. Solid Free. Fabr. Symp., Austin, TEXAS, 1997,25.

[8] J. E. Smay, J. Cesarano, J. A. Lewis, Langmuir 2002, 18, 5429.

[9] S. Michna, W. Wu, J. A. Lewis, Biomaterials 2005, 26, 5632.

[10] A. Kumar, A. R. Akkineni, B. Basu, M. Gelinsky, **2016**, *30*, 1168.

[11] P. Miranda, A. Pajares, E. Saiz, A. P. Tomsia, F. Guiberteau, J. Biomed. Mater. Res. A 2008, 85, 218.

[12] P. Miranda, E. Saiz, K. Gryn, A. P. Tomsia, Acta Biomater. 2006, 2, 457.

[13] Y. Zhang, L. Xia, D. Zhai, M. Shi, Y. Luo, C. Feng, B. Fang, J. Yin, J. Chang, C. Wu, Nanoscale 2015, 7, 19207.

[14] M. Houmard, Q. Fu, M. Genet, E. Saiz, A. P. Tomsia, J. Biomed. Mater. Res. - Part B Appl. Biomater. 2013, 101, 1233.

[15] C. Mangano, B. Barboni, L. Valbonetti, P. Berardinelli, A. Martelli, A. Muttini, R. Bedini, S. Tete, A. Piattelli, M. Mattioli, J. Oral Implantol. 2015, 41, 240.

[16] J. Franco, P. Hunger, M. E. Launey, A. P. Tomsia, E. Saiz, Acta Biomater. 2010, 6, 218.

[17] S. Ishack, A. Mediero, T. Wilder, J. L. Ricci, B. N. Cronstein, J. Biomed. Mater. Res. - Part 8 Appl. Biomater. 2017, 105, 366.

[18] Q. Fu, E. Saiz, A. P. Tomsia, Adv. Funct. Mater. 2011, 21, 1058.

[19] X. Liu, M. N. Rahaman, G. E. Hilmas, B. S. Bal, Acta Biomater. 2013, 9, 7025.

[20] Y. Luo, A. Lode, C. Wu, M. Gelinsky, Adv. Bioact. Inorg. Mater. Bone Regen. Drug Deliv. 2013, 83.

[21] C. Wu, Y. Luo, G. Cuniberti, Y. Xiao, M. Gelinsky, Acta Biomater. 2011, 7, 2644.

[22] A. Motealleh, S. Eqtesadi, F. H. Perera, A. Pajares, F. Guiberteau, P. Miranda, J. Mech. Behav. Biomed. Mater. 2016, 64, 253.

[23] H. Yun, S. Kim, Y. Hyeon, Chem. Commun. 2007, 2139.

[24] S. Eqtesadi, A. Motealleh, P. Miranda, A. Lemos, A. Rebelo, J. M. F. Ferreira, Mater. Lett. 2013, 93, 68.

[25] A. V. Lluch, A. C. Fernandez, G. G. Ferrer, M. M. Pradas, J. Biomed. Mater. Res. B. Appl. Biomater. 2009, 90, 182.

[26] D. J. Hoelzle, A. G. Alleyne, A. J. Wagoner Johnson, Acta Biomater. 2008, 4, 897.

[27] C. Wu, W. Fan, Y. Zhou, Y. Luo, M. Gelinsky, J. Chang, Y. Xiao, J. Mater. Chem. 2012, 22, 12288.

[28] M. M. Farag, H. Yun, Mater. Lett. 2014, 132, 111.

[29] Y. Maazouz, E. B. Montufar, J. Guillem-Marti, I. Fleps, C. Ohman, C. Persson, M. P. Ginebra, J. Mater. Chem. 6 2014, 2, 5378.

[30] A. Lode, K. Meissner, Y. Luo, F. Sonntag, S. Glorius, B. Nies, C. Vater, F. Despang, T. Hanke, M. Gelinsky, J. Tissue Eng. Regen. Med. 2014, 8, 682.

[31] W. J. Kim, H.-S. Yun, G. H. Kim, Sci. Rep. 2017, 7, 3181.

[32] N. Raja, H. Yun, J. Mater. Chem. B 2016, 4, 4707.

[33] Y. Luo, A. Lode, F. Sonntag, B. Nies, M. Gelinsky, J. Mater. Chem. B 2013, 1, 4088.

[34] A. R. Akkineni, Y. Luo, M. Schumacher, B. Nies, A. Lode, M. Gelinsky, Acta Biomater. 2015, 27, 264.

[35] D. Pastorino, C. Canal, M.P. Ginebra, Acta Biomater. 2015, 28, 205.

**Claims**

1. A 3D-printed synthetic bone graft with a composition comprising calcium-deficient hydroxyapatite (CDHA) in an amount of at least 60 wt.% with respect to the total weight of the composition, optionally $\alpha$-TCP and/or $\beta$-TCP, and having a binder content in the range of 5 wt.% or less, preferably in the range of 0.25 wt.% to 1 wt.% with respect to the total weight of the composition, wherein the composition has a macro-porosity in the range of 10 % to 60%, as determined by calculating the difference between the total porosity and the nano-micro porosity, wherein

    the nano-microporosity is determined by mercury intrusion porosimetry; and
    the macroporosity is defined as a pore entrance size larger than 10 $\mu$m, the pore entrance size being determined by mercury intrusion porosimetry.

2. The synthetic bone graft according to claim 1, wherein the calcium-deficient hydroxyapatite is doped with one or more ions selected from the group consisting of anions such as carbonate, bicarbonate, silicate, and/or cations such as Ca, Mg, Sr, Ce, Al, Zn, Ag, Co, Cu and other transition metals.

3. Synthetic bone graft according to claim 1 or 2, wherein the composition contains more than 80 wt.% calcium-deficient hydroxyapatite (CDHA).

4. Synthetic bone graft according to one or more of the preceding claims, wherein the binder is a poly(oxypropylene)-poly(oxyethylene) copolymer, such as poloxamer, preferably poloxamer 407.

5. Synthetic bone graft according to one or more of the preceding claims, wherein the composition of the synthetic bone graft has a specific surface area (SSA) in the range of 10 to 15 $m^2/g$.

6. Synthetic bone graft according to one or more of the preceding claims, wherein the composition has a total porosity in the range of 20 % to 80%, as determined by mercury intrusion porosimetry.

7. Synthetic bone graft according to one or more of the preceding claims, wherein the composition has pores of less than 1 $\mu$m, as determined by mercury intrusion porosimetry.

8. Synthetic bone graft according to one or more of the preceding claims, wherein the composition has a density in the range of 2.70 to 3.14 $g/cm^3$, as determined by helium pycnometry.

9. Use of a self-setting ink for the manufacture of synthetic bone graft in the form of a 3D-printed scaffold, wherein the self-setting ink comprises $\alpha$-TCP powder and a binder solution comprising a poloxamer and water, wherein the binder solution contains 25g to 60 g poloxamer per 100 ml solution.

10. The use according to claim 9, wherein the binder solution contains from 25g to 35g poloxamer per 100 ml solution.

11. The use according to any one of claims 9 to 10, wherein 0.3 to 0.7 g binder solution is used per g of $\alpha$-TCP powder.

12. Method for producing a synthetic bone graft according to one or more of claims 1 to 8, comprising the following steps:

a) Providing a self-setting ink comprising $\alpha$-TCP powder and a binder solution comprising a poloxamer and water;
b) 3D printing to provide scaffold;
c) placing the scaffold in a humid atmosphere; and
d) hardening the scaffold by immersion in water or in an aqueous solution consisting of water and one or more ions selected from the group consisting of anions such as carbonate, bicarbonate, silicate, and/or cations such as Ca, Mg, Sr, Ce, Al, Zn, Ag, Co, Cu and other transition metals.

13. Method according to claim 12, wherein hardening step d) is performed by immersing the scaffold in water and autoclaving at a temperature above 100 °C and a pressure above 1 atm of absolute pressure, preferably wherein hardening step d) is performed in 120 minutes or less.

14. Method according to claim 12 or 13, wherein in step b) the 3D printing process is performed to provide a shape that was pre-determined by a digitalised medical imaging technique and/or computer aided design.

**Patentansprüche**

1. 3D-gedrucktes synthetisches Knochenimplantat mit einer Zusammensetzung, die calciumdefizientes Hydroxyapatit (CDHA) in einer Menge von mindestens 60 Gew.-%, bezogen auf das Gesamtgewicht der Zusammensetzung, optional $\alpha$-TCP und/oder $\beta$-TCP, umfasst und einen Bindemittelgehalt im Bereich von 5 Gew.-% oder weniger, vorzugsweise im Bereich von 0,25 Gew.-% bis 1 Gew.-%, bezogen auf das Gesamtgewicht der Zusammensetzung, aufweist, wobei die Zusammensetzung eine Makro-Porosität im Bereich von 10% bis 60 % aufweist, wie durch Berechnen der Differenz zwischen der Gesamtporosität und der Nano-Mikro-Porosität bestimmt, wobei

die Nano-Mikro-Porosität durch Quecksilber-Intrusionsporosimetrie bestimmt wird; und
die Makro-Porosität als eine Poreneintrittsgröße größer als 10 $\mu$m definiert ist, wobei die Poreneintrittsgröße durch Quecksilber-Intrusionsporosimetrie bestimmt wird.

2. Synthetisches Knochenimplantat nach Anspruch 1, wobei das calciumdefiziente Hydroxyapatit mit einem oder mehreren Ionen dotiert ist, die aus der Gruppe bestehend aus Anionen wie Carbonat, Hydrogencarbonat, Silikat und/oder Kationen wie Ca, Mg, Sr, Ce, Al, Zn, Ag, Co, Cu und anderen Übergangsmetallen ausgewählt sind.

3. Synthetisches Knochenimplantat nach Anspruch 1 oder 2, wobei die Zusammensetzung mehr als 80 Gew.-% calciumdefizientes Hydroxyapatit (CDHA) enthält.

4. Synthetisches Knochenimplantat nach einem oder mehreren der vorhergehenden Ansprüche, wobei das Bindemittel ein Poly(oxypropylen)-poly(oxyethylen)-Copolymer, wie Poloxamer, vorzugsweise Poloxamer 407, ist.

**5.** Synthetisches Knochenimplantat nach einem oder mehreren der vorhergehenden Ansprüche, wobei die Zusammensetzung des synthetischen Knochenimplantats eine spezifische Oberfläche (specific surface area - SSA) im Bereich von 10 bis 15 m$^2$/g aufweist.

**6.** Synthetisches Knochenimplantat nach einem oder mehreren der vorhergehenden Ansprüche, wobei die Zusammensetzung eine Gesamtporosität im Bereich von 20 % bis 80 % aufweist, wie durch Quecksilber-Intrusionsporosimetrie bestimmt.

**7.** Synthetisches Knochenimplantat nach einem oder mehreren der vorhergehenden Ansprüche, wobei die Zusammensetzung Poren von weniger als 1 $\mu$m aufweist, wie durch Quecksilber-Intrusionsporosimetrie bestimmt.

**8.** Synthetisches Knochenimplantat nach einem oder mehreren der vorhergehenden Ansprüche, wobei die Zusammensetzung eine Dichte im Bereich von 2,70 bis 3,14 g/cm$^3$ aufweist, wie durch Helium-Pyknometrie bestimmt.

**9.** Verwendung einer selbsthärtenden Tinte zur Herstellung von synthetischem Knochenimplantat in Form eines 3D-gedruckten Gerüsts, wobei die selbsthärtende Tinte $\alpha$-TCP-Pulver und eine Bindemittellösung umfassend ein Poloxamer und Wasser umfasst, wobei die Bindemittellösung 25 g bis 60 g Poloxamer pro 100 ml Lösung enthält.

**10.** Verwendung nach Anspruch 9, wobei die Bindemittellösung 25 g bis 35 g Poloxamer pro 100 ml Lösung enthält.

**11.** Verwendung nach einem der Ansprüche 9 bis 10, wobei 0,3 bis 0,7 g Bindemittellösung pro g $\alpha$-TCP-Pulver verwendet wird.

**12.** Verfahren zum Herstellen eines synthetischen Knochenimplantats nach einem oder mehreren der Ansprüche 1 bis 8, die folgenden Schritte umfassend:

a) Bereitstellen einer selbsthärtenden Tinte, umfassend ein $\alpha$-TCP-Pulver und eine Bindemittellösung, umfassend ein Poloxamer und Wasser;
b) 3D-Drucken, um ein Gerüst bereitzustellen;
c) Platzieren des Gerüsts in einer feuchten Atmosphäre; und
d) Härten des Gerüsts durch Eintauchen in Wasser oder in eine wässrige Lösung, bestehend aus Wasser und einem oder mehreren Ionen, ausgewählt aus der Gruppe bestehend aus Anionen wie Carbonat, Hydrogencarbonat, Silikat und/oder Kationen wie Ca, Mg, Sr, Ce, Al, Zn, Ag, Co, Cu und anderen Übergangsmetallen.

**13.** Verfahren nach Anspruch 12, wobei der Schritt des Härtens d) durch Eintauchen des Gerüsts in Wasser und Autoklavieren bei einer Temperatur oberhalb von 100 °C und einem Druck oberhalb von 1 atm absoluten Drucks durchgeführt wird, vorzugsweise wobei der Schritt des Härtens d) in 120 Minuten oder weniger durchgeführt wird.

**14.** Verfahren nach Anspruch 12 oder 13, wobei in Schritt b) der 3D-Druck-Prozess durchgeführt wird, um eine Form bereitzustellen, die durch eine digitalisierte medizinische Bildgebungstechnik und/oder computergestütztes Design vorgegeben wurde.

**Revendications**

**1.** Greffe osseuse synthétique imprimée en 3D avec une composition comprenant de l'hydroxyapatite déficiente en calcium (CDHA) en une quantité d'au moins 60 % en poids par rapport au poids total de la composition, éventuellement de l'$\alpha$-TCP et/ou du $\beta$-TCP, et ayant une teneur en liant dans la plage de 5 % en poids ou moins, de préférence dans la plage de 0,25 % en poids à 1 % en poids par rapport au poids total de la composition, dans laquelle la composition a une macro-porosité dans la plage de 10 % à 60 %, telle que déterminée par calcul de la différence entre la porosité totale et la nano-microporosité, dans laquelle

la nano-microporosité est déterminée par porosimétrie par intrusion de mercure ; et
la macroporosité est définie comme une taille d'entrée de pore supérieure à 10 $\mu$m, la taille d'entrée de pore étant déterminée par porosimétrie par intrusion de mercure.

**2.** Greffe osseuse synthétique selon la revendication 1, dans laquelle l'hydroxyapatite déficiente en calcium est dopée par un ou plusieurs ions choisis dans le groupe constitué par des anions tels que carbonate, bicarbonate, silicate et/ou des cations tels que Ca, Mg, Sr, Ce, Al, Zn, Ag, Co, Cu et autres métaux de transition.

3. Greffe osseuse synthétique selon la revendication 1 ou 2, dans laquelle la composition contient plus de 80 % en poids d'hydroxyapatite déficiente en calcium (CDHA).

4. Greffe osseuse synthétique selon une ou plusieurs des revendications précédentes, dans laquelle le liant est un copolymère poly(oxypropylène)-poly(oxyéthylène), tel qu'un poloxamère, de préférence le poloxamère 407.

5. Greffe osseuse synthétique selon une ou plusieurs des revendications précédentes, dans laquelle la composition de la greffe osseuse synthétique présente une surface spécifique (SSA) dans la plage de 10 à 15 $m^2/g$.

6. Greffe osseuse synthétique selon une ou plusieurs des revendications précédentes, dans laquelle la composition présente une porosité totale dans la plage de 20 % à 80 %, telle que déterminée par porosimétrie par intrusion de mercure.

7. Greffe osseuse synthétique selon une ou plusieurs des revendications précédentes, dans laquelle la composition présente des pores inférieurs à 1 $\mu$m, tels que déterminés par porosimétrie par intrusion de mercure.

8. Greffe osseuse synthétique selon une ou plusieurs des revendications précédentes, dans laquelle la composition a une masse volumique dans la plage de 2,70 à 3,14 $g/cm^3$, telle que déterminée par pycnométrie à l'hélium.

9. Utilisation d'une encre auto-durcissable pour la fabrication d'une greffe osseuse synthétique sous la forme d'un échafaudage imprimé en 3D, dans laquelle l'encre auto-durcissable comprend de la poudre d'$\alpha$-TCP et une solution de liant comprenant un poloxamère et de l'eau, dans laquelle la solution de liant contient 25 g à 60 g de poloxamère par 100 ml de solution.

10. Utilisation selon la revendication 9, dans laquelle la solution de liant contient de 25 g à 35 g de poloxamère par 100 ml de solution.

11. Utilisation selon l'une quelconque des revendications 9 et 10, dans laquelle 0,3 à 0,7 g de solution de liant est utilisé par g de poudre d'$\alpha$-TCP.

12. Procédé de fabrication d'une greffe osseuse synthétique selon une ou plusieurs des revendications 1 à 8, comprenant les étapes suivantes :

   a) la fourniture d'une encre auto-durcissable comprenant de la poudre d'$\alpha$-TCP et une solution de liant comprenant un poloxamère et de l'eau ;
   b) l'impression 3D pour fournir un échafaudage ;
   c) le placement de l'échafaudage dans une atmosphère humide ; et
   d) le durcissement de l'échafaudage par immersion dans de l'eau ou dans une solution aqueuse constituée d'eau et d'un ou plusieurs ions choisis dans le groupe constitué par des anions tels que carbonate, bicarbonate, silicate et/ou des cations tels que Ca, Mg, Sr, Ce, Al, Zn, Ag, Co, Cu et d'autres métaux de transition.

13. Procédé selon la revendication 12, dans lequel l'étape d) de durcissement est réalisée par immersion de l'échafaudage dans de l'eau et passage dans un autoclave à une température supérieure à 100 °C et une pression supérieure à 1 atm de pression absolue, de préférence dans lequel l'étape d) de durcissement est réalisée en 120 minutes ou moins.

14. Procédé selon la revendication 12 ou 13, dans lequel à l'étape b), le procédé d'impression 3D est réalisé pour donner une forme prédéterminée par une technique d'imagerie médicale numérisée et/ou une conception assistée par ordinateur.

**FIGURES**

**FIG. 1**

| Self-setting ink | | 3D printing | | | | Hardening process | |
|---|---|---|---|---|---|---|---|
| Prepare the binder solution | Mix the solid and liquid phase | Design scaffold from medical images | Configure the printer software | Prepare the 3D printer, introduce ink | Ink deposition | Pre-setting in a humid atmosphere | Setting immersed in water |

**FIG. 2**

a) 260 μm strand | 311 μm strand | 413 μm strand

b) 40 % infill | 50 % infill | 60 % infill | 70 % infill

c) 60 % infill | 70 % infill | 80 % infill | 90 % infill

**FIG. 3**

## FIG. 4

### a) Hardening process

| Biomimetic | Hydrothermal Immersed | Hydrothermal Vapour |
|---|---|---|

37°C, 6 days 1 atm. (P$_{atm}$)

Autoclave 121°C, 30 min 2 atm

Autoclave 121°C, 100 min 2 atm

### b) XRD

■ α-TCP    ▯ β-TCP    ▲ CDHA
—— Fit curve of α-TCP    – – Fit curve of β-TCP    - - - Fit curve of CDHA

### c) SEM

### d) SSA

| 12.345 m2/g | 11.664 m2/g | 2.342 m2/g |
|---|---|---|

### e) MIP

—— Biomimetic    – – Hydrothermal-Vapour    - - - Hydrothermal-Immersed

**FIG. 4 (CONT.)**

f) Porosity

g) Compressive strength

h) TGA

**FIG. 5**

(a)

(b)

(c)

(d)

**FIG. 6**

a)

b)

c)

d)

e)

FIG. 7

**FIG. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MAAZOUZ et al.** *acta biomaterialia,* vol. 49, 563-574
- **C. G. FINKEMEIER.** *J. Bone Joint Surg. Am.,* 2002, vol. 84-A, 454
- **M. BOHNER.** *Mater. Today,* 2010, vol. 13, 24
- **B. STEVENS ; Y. YANG ; A. MOHANDAS ; B. STUCKER ; K. T. NGUYEN.** *J. Biomed. Mater. Res. Part 8 Appl. Biomater.,* 2008, vol. 858, 573
- **S. BOSE ; S. VAHABZADEH ; A. BANDYOPADHYAY.** *Mater. Today,* 2013, vol. 16, 496
- **D. TANG ; R. S. TARE ; L. Y. YANG ; D. F. WILLIAMS ; K. L. OU ; R. O. C. OREFFO.** *Biomaterials,* 2016, vol. 83, 363
- **A. BUTSCHER ; M. BOHNER ; S. HOFMANN ; L. GAUCKLER ; R. MÜLLER.** *Acta Biomater.,* 2011, vol. 7, 907
- **J. CESARANO ; T. A. BAER ; P. CALVERT.** *Proc. Solid Free. Fabr. Symp., Austin, TEXAS,* 1997, 25
- **J. E. SMAY ; J. CESARANO ; J. A. LEWIS.** *Langmuir,* 2002, vol. 18, 5429
- **S. MICHNA ; W. WU ; J. A. LEWIS.** *Biomaterials,* 2005, vol. 26, 5632
- **P. MIRANDA ; A. PAJARES ; E. SAIZ ; A. P. TOMSIA ; F. GUIBERTEAU.** *J. Biomed. Mater. Res. A,* 2008, vol. 85, 218
- **P. MIRANDA ; E. SAIZ ; K. GRYN ; A. P. TOMSIA.** *Acta Biomater.,* 2006, vol. 2, 457
- **Y. ZHANG ; L. XIA ; D. ZHAI ; M. SHI ; Y. LUO ; C. FENG ; B. FANG ; J. YIN ; J. CHANG ; C. WU.** *Nanoscale,* 2015, vol. 7, 19207
- **M. HOUMARD ; Q. FU ; M. GENET ; E. SAIZ ; A. P. TOMSIA.** *J. Biomed. Mater. Res. - Part B Appl. Biomater.,* 2013, vol. 101, 1233
- **C. MANGANO ; B. BARBONI ; L. VALBONETTI ; P. BERARDINELLI ; A. MARTELLI ; A. MUTTINI ; R. BEDINI ; S. TETE ; A. PIATTELLI ; M. MATTIOLI.** *J. Oral Implantol.,* 2015, vol. 41, 240
- **J. FRANCO ; P. HUNGER ; M. E. LAUNEY ; A. P. TOMSIA ; E. SAIZ.** *Acta Biomater.,* 2010, vol. 6, 218
- **S. ISHACK ; A. MEDIERO ; T. WILDER ; J. L. RICCI ; B. N. CRONSTEIN.** *J. Biomed. Mater. Res. - Part 8 Appl. Biomater.,* 2017, vol. 105, 366
- **Q. FU ; E. SAIZ ; A. P. TOMSIA.** *Adv. Funct. Mater.,* 2011, vol. 21, 1058
- **X. LIU ; M. N. RAHAMAN ; G. E. HILMAS ; B. S. BAL.** *Acta Biomater.,* 2013, vol. 9, 7025
- **Y. LUO ; A. LODE ; C. WU ; M. GELINSKY.** *Adv. Bioact. Inorg. Mater. Bone Regen. Drug Deliv.,* 2013, 83
- **C. WU ; Y. LUO ; G. CUNIBERTI ; Y. XIAO ; M. GELINSKY.** *Acta Biomater.,* 2011, vol. 7, 2644
- **A. MOTEALLEH ; S. EQTESADI ; F. H. PERERA ; A. PAJARES ; F. GUIBERTEAU ; P. MIRANDA.** *J. Mech. Behav. Biomed. Mater.,* 2016, vol. 64, 253
- **H. YUN ; S. KIM ; Y. HYEON.** *Chem. Commun.,* 2007, 2139
- **S. EQTESADI ; A. MOTEALLEH ; P. MIRANDA ; A. LEMOS ; A. REBELO ; J. M. F. FERREIRA.** *Mater. Lett.,* 2013, vol. 93, 68
- **A. V. LLUCH ; A. C. FERNANDEZ ; G. G. FERRER ; M. M. PRADAS.** *J. Biomed. Mater. Res. B. Appl. Biomater.,* 2009, vol. 90, 182
- **D. J. HOELZLE ; A. G. ALLEYNE ; A. J. WAGONER JOHNSON.** *Acta Biomater.,* 2008, vol. 4, 897
- **C. WU ; W. FAN ; Y. ZHOU ; Y. LUO ; M. GELINSKY ; J. CHANG ; Y. XIAO.** *J. Mater. Chem.,* 2012, vol. 22, 12288
- **M. M. FARAG ; H. YUN.** *Mater. Lett.,* 2014, vol. 132, 111
- **Y. MAAZOUZ ; E. B. MONTUFAR ; J. GUILLEM-MARTI ; I. FLEPS ; C. OHMAN ; C. PERSSON ; M. P. GINEBRA.** *J. Mater. Chem.,* 2014, vol. 2, 5378
- **A. LODE ; K. MEISSNER ; Y. LUO ; F. SONNTAG ; S. GLORIUS ; B. NIES ; C. VATER ; F. DESPANG ; T. HANKE ; M. GELINSKY.** *J. Tissue Eng. Regen. Med.,* 2014, vol. 8, 682
- **W. J. KIM ; H.-S. YUN ; G. H. KIM.** *Sci. Rep.,* 2017, vol. 7, 3181
- **N. RAJA ; H. YUN.** *J. Mater. Chem. B,* 2016, vol. 4, 4707
- **Y. LUO ; A. LODE ; F. SONNTAG ; B. NIES ; M. GELINSKY.** *J. Mater. Chem. B,* 2013, vol. 1, 4088
- **A. R. AKKINENI ; Y. LUO ; M. SCHUMACHER ; B. NIES ; A. LODE ; M. GELINSKY.** *Acta Biomater.,* 2015, vol. 27, 264
- **D. PASTORINO ; C. CANAL ; M.P. GINEBRA.** *Acta Biomater.,* 2015, vol. 28, 205